(19) Europäisches Patentamt / European Patent Office / Office européen des brevets

(11) **EP 3 227 813 B1**

(12) **FASCICULE DE BREVET EUROPEEN**

(45) Date de publication et mention
de la délivrance du brevet:
**12.02.2020 Bulletin 2020/07**

(21) Numéro de dépôt: **15808766.8**

(22) Date de dépôt: **30.11.2015**

(51) Int Cl.:
***G16B 25/20*** *(2019.01)*

(86) Numéro de dépôt international:
**PCT/FR2015/053256**

(87) Numéro de publication internationale:
**WO 2016/087756 (09.06.2016 Gazette 2016/23)**

(54) **PROCÉDÉ D'ESTIMATION DE L'AFFINITÉ SONDE-CIBLE D'UNE PUCE A ADN ET PROCÉDÉ DE FABRICATION D'UNE PUCE A ADN**

VERFAHREN ZUR KALKULATION DER SONDEN-ZIEL-AFFINITÄT VON EINEM DNA-CHIP SOWIE VERFAHREN ZUR HERSTELLUNG EINES DNA-CHIPS

METHOD FOR ESTIMATING THE PROBE-TARGET AFFINITY OF A DNA CHIP AND METHOD FOR MANUFACTURING A DNA CHIP

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.12.2014 FR 1461722**

(43) Date de publication de la demande:
**11.10.2017 Bulletin 2017/41**

(73) Titulaires:
• **Biomerieux**
**69280 Marcy-l'Étoile (FR)**
• **Hospices Civils De Lyon**
**69002 Lyon (FR)**

(72) Inventeurs:
• **BECKER, Jérémy**
**28000 Chartres (FR)**
• **PEROT, Philippe**
**16710 Saint-Yrieix (FR)**
• **MALLET, François**
**69100 Villeurbanne (FR)**

(74) Mandataire: **Regimbeau**
**20, rue de Chazelles**
**75847 Paris Cedex 17 (FR)**

(56) Documents cités:
**US-A1- 2005 026 176      US-A1- 2005 250 115
US-A1- 2013 338 015**

EP 3 227 813 B1

**Description**

L'invention a trait au domaine de la transcriptomique, notamment de l'étude l'hybridation entre des brins d'ADN.

L'invention trouve particulièrement application dans le domaine de la conception de supports d'hybridation, notamment de puces à ADN.

## ETAT DE LA TECHNIQUE

Une puce à ADN mesure le niveau d'expression de transcrits en se fondant sur la propriété qu'a un simple brin d'ADN à reformer spontanément un double brin lorsqu'il est mis en présence d'un brin d'ADN complémentaire, c'est-à-dire la propriété qu'il a à s'hybrider avec un brin complémentaire. Pour connaitre le niveau d'expression d'un transcrit dans un échantillon biologique, une puce à ADN comprend des séquences de bases azotées, appelées « sondes », conçues pour s'hybrider de manière spécifique avec un ensemble de transcrits d'intérêts, ou transcrits « cibles ». Pour améliorer la robustesse de la mesure, un transcrit est ciblé par plusieurs sondes, formant ensemble un « probe set ». Aux fins de screening à haut débit, une puce à ADN comprend ainsi $I$ « probe sets » ciblant $I$ transcrits, pour une totalité de $J$ sondes différentes. Aux fins de mesure, chaque sonde est répétée à l'identique un grand nombre de fois, les sondes répétées étant disposées dans un puit.

Le transcrit cible dont on cherche l'expression, qui peut faire plusieurs milliers ou dizaines de milliers de bases azotées A, G, C, T, est tout d'abord transformé, via un processus d'amplification, en une solution comprenant des fragments d'ADN plus petits, de longueur comprise usuellement entre 25 et 200 bases azotées, marqués par un colorant fluorescent. La solution ainsi obtenue est alors déposée dans les puits de la puce à ADN. Chaque puits correspondant à une sonde, répétée plusieurs fois et conçue pour un transcrit, ceci mène donc à l'hybridation de certains de ces fragments avec les sondes du puits. Après un lavage de la puce à ADN afin de ne conserver que les hybrides formés dans les puits, une mesure de la fluorescence dans chaque puits est alors mise en œuvre par un scanner haute résolution, mesure représentative de la quantité d'hybrides présente dans le puits. On parle alors de la « fluorescence de la sonde », ou « d'intensité de la sonde ».

Pour bien comprendre ce qui suit, il convient d'introduire les définitions suivantes. Le terme de « sonde » se réfère donc à une séquence de bases azotées, ou « nucléotides », constitutive d'une puce à ADN, et de manière plus générale à tout dispositif utilisant l'hybridation avec des sondes. Le terme de « cible » se réfère à une séquence de bases azotées, issue d'un transcrit, apte à former avec sa sonde un hybride. L'expression de « cible spécifique » se rapporte à une cible qui correspond à la portion de transcrit identifiée, tant en termes de séquence de base que de position dans le transcrit, pour laquelle la sonde a été conçue. Le terme d'hybride « parfait » ou « identique » se rapporte à un hybride formé d'une sonde et d'une cible qui sont des complémentaires strictes l'un de l'autre en termes de bases azotées (hybride plus connu sous l'expression anglaise de « perfect match »). L'expression « défaut d'appariement » se rapporte à un hybride d'une sonde et d'une cible dans lequel une base de la sonde et une base de la cible l'une en face de l'autre ne sont pas complémentaires (plus connu sous le terme anglais de « missmatch ») ou à une base de la cible ou de la sonde qui n'est en face d'aucune base (plus connu sous le terme anglais de « gap »). On parlera également d'une sonde et d'une cible ayant un défaut d'appariement. Le terme de « k-mer » se rapporte à une séquence de k bases nucléiques. La « longueur » d'une séquence de base azotée correspond au nombre de bases azotées qu'elle contient. La longueur d'un hybride sonde/cible correspond le plus généralement à la longueur de la sonde.

Si le principe général d'une puce à ADN semble simple a priori, puisqu'il consiste à choisir une sonde qui correspond à une séquence d'ADN complémentaire d'un fragment du transcrit, sa mise en pratique pour obtenir des puces à ADN de qualité est cependant délicate.

En effet, de prime abord, on peut penser qu'il suffit de simplement choisir une sonde qui forme avec une cible un hybride parfait. Or, un hybride parfait peut être trop instable pour résister au lavage, ce qui mène au final à un signal mesuré trop faible pour déterminer le niveau d'expression du transcrit. On note ainsi que pour un transcrit donné, les portions de celui-ci donnant lieu aux sondes ne se valent pas, et qu'il convient donc de choisir les portions de transcrit menant à l'obtention d'hybrides sonde/cible suffisamment stables pour obtenir une mesure significative. En outre, une sonde et une cible, pouvant présenter un ou plusieurs défauts d'appariement, peuvent également s'hybrider de manière stable. Une telle cible, pouvant être différente de la cible spécifique, peut provenir d'un autre transcrit présent dans l'échantillon biologique, auquel cas il est obtenu une fausse détection ou « faux positif ».

C'est pourquoi, il est recherché une sonde qui, à la fois :

- ne vise qu'une seule portion déterminée du transcrit, portion qui est unique, et ne se retrouve donc pas à l'identique dans le transcrit lui-même à une autre position ou dans un autre transcrit susceptible d'être présent dans l'échantillon biologique, et présente une faible affinité avec toute autre cible présentant un défaut d'appariement. On parle alors de « sonde spécifique » ; et
- présente une forte affinité avec la cible spécifique, c'est-à-dire forme un hybride stable avec celle-ci. On parle alors d'une « forte affinité de la sonde avec la cible spécifique», ou de sonde « affine » ou « sensible ».

Sachant qu'un transcrit peut comporter des dizaines de milliers de bases, et qu'un échantillon biologique peut comprendre beaucoup de transcrits, sans que l'on puisse aisément et efficacement contrôler la composition de l'échantillon, on comprend aisément le nombre de sondes, de longueur inférieure à la centaine de bases, qu'il faudrait concevoir et tester pour ne retenir que les sondes spécifiques et affines. La conception expérimentale d'une puce à ADN étant donc difficile, voire impossible, des outils bioinformatiques ont donc été conçus pour évaluer la spécificité et l'affinité des sondes et ainsi aider à la conception d'une puce à ADN.

Par exemple, le document de Mei et al (« Probe selection for high-density oligonucleotide arrays », Proceedings of the National Academy of Sience, 100(20) :11237-11242, septembre 2003) décrit un score quantifiant la qualité d'une sonde. Ce score comprend le produit d'un premier terme, quantifiant la spécificité de la sonde, par un deuxième terme dérivé de l'affinité de la sonde. Le premier terme détermine le risque de réactions croisée entre la sonde et d'autres transcrits, différents du transcrit cible, sur l'ensemble du génome humain. Le second terme comprend quant à lui un terme de sensibilité, exprimé comme le coefficient directeur de la droite I = K + S.ln([T]), où I est l'intensité d'une sonde donnée, S la sensibilité, K l'affinité cible-sonde et [T] la concentration de cible. Les auteurs montrent que ce terme de sensibilité peut être calculé à l'aide d'un modèle d'hybridation basé sur la séquence nucléique des sondes. Selon ce modèle, la sonde est donc considérée, du point de vue de l'affinité, uniquement comme une juxtaposition de bases sans lien particulier entre elles, ni lien avec une cible avec laquelle elle peut s'hybrider. Ce modèle d'affinité, parfois appelé « modèle monomère toute position », est donc exprimé uniquement en fonction de la présence/absence de chaque base à chaque position.

Ce modèle d'affinité s'avérant toutefois peu satisfaisants car où il ne tient pas compte de défauts d'appariement entre une sonde et une cible, et cherche uniquement à modéliser l'affinité d'une sonde avec sa cible spécifique. Des modèles d'affinité plus complexes ont donc été conçus pour tenir compte des hybridations non-spécifique, ou réactions croisées. Par exemple, le document Zhang et al (« A model of molecular interactions on short oligonucleotide microarrays », Nature biotechnology, 21(7) :818-821, juillet 2003) décrit un modèle statistique exprimant l'intensité mesurée comme la somme de deux termes liés respectivement à l'hybridation de la sonde avec sa cible spécifique et l'hybridation de la sonde avec l'ensemble des cibles non spécifiques. Dans ce document, l'intensité du $i^{ème}$ puit de la puce à ADN, c'est-à-dire de la $i^{ème}$ sonde, reflétant le niveau d'expression du $j^{ème}$ transcrit, est ainsi prédite selon les relations :

$$\hat{I}_{ij} = \frac{N_j}{(1 + e^{E_{ij}})} + \frac{N_j^{\star}}{(1 + e^{E_{ij}^{\star}})} + B$$

$$E_{ij} = \sum_k \omega_k \cdot \epsilon(b_k, b_{k+1})$$

$$E_{ij}^{\star} = \sum_k \omega_k^{\star} \cdot \epsilon^{\star}(b_k, b_{k+1})$$

où :

- $\hat{I}_{ij}$ est l'intensité prédite ;
- $N_j$ est la quantité de cibles, issus du $j^{ème}$ transcrit, mesuré par le $j^{ème}$ probe sets, présente dans la solution déposée sur la puce à ADN ;
- $\widehat{N}_j^{\star}$ est la quantité de cibles, issue de transcrits différents du $j^{ème}$ transcrit ciblé par les sondes du probe set, présente dans la solution déposée sur la puce à ADN ;
- $\varepsilon(b_k, b_{k+1})$ est l'énergie de libération du couple $(b_k, b_{k+1})$ de bases consécutives de la sonde, ou « dimère », présentes respectivement à la position $k$ et $k+1$ dans la sonde, indépendamment de la position dudit couple dans la sonde, énergie de libération du couple lorsque la sonde est hybridé avec sa cible spécifique, et donc forme avec cette cible un hybride parfait ;
- $\omega_k$ est un facteur quantifiant l'influence de la position du couple $(b_k, b_{k+1})$ dans la sonde lorsque la sonde est hybridée avec cette cible ;
- $\varepsilon^{\star}(b_k, b_{k+1})$ est une énergie de libération du couple $(b_k, b_{k+1})$, indépendamment de la position de celui-ci dans la sonde, énergie de libération du couple lorsque la sonde est hybridée avec une cible issue d'un autre transcrit, et donc pouvant comporter un défaut d'appariement ; et

- $\omega_k^\star$ est un facteur quantifiant l'influence de la position du couple $(b_k, b_{k+1})$ dans la sonde lorsque cette dernière est hybridée avec cette cible ; et

- $B$ est un scalaire.

La valeur de ces paramètres est déterminée en mettant en œuvre une identification sur la base d'intensités mesurées par les auteurs du document.

Le premier terme

$$\frac{N_j}{(1 + e^{E_{kj}})},$$

correspondant à la contribution de la cible spécifique est égal au quotien de la quantité de fragments spécifiques hybridé sur la puce à ADN et d'un terme reflétant l'affinité entre la sonde et sa cible spécifique, à savoir un terme exponentiel à l'énergie nécessaire pour séparer la sonde de sa cible spécque . Selon le modèle, l'énergie est égale à la somme des contributions respectives des dimères $(b_k, b_{k+1})$ pondérées par des termes dépendant de la position des dimères. Par définition, ce terme provient donc exclusivement d'hybrides parfaits formés dans le probe set. Pour chaque dimère, il n'existe donc qu'une unique configuration, à savoir la liaison à son complémentaire.

Le second terme

$$\frac{N_j^\star}{(1 + e^{E_{kj}^\star})}$$

correspond quant à lui à la proportion des sondes non spécifiques. Or, à la différence d'une sonde hybridée avec sa cible spécifique, lorsque l'on considère l'hybridation avec des cibles d'autres transcrits, c'est-à-dire les hybridations croisées ou « cross-hybridation », il peut certes exister une hybridation parfaite (si un autre transcrit donne lieu à une cible identique à la cible spécifique) mais il existe surtout une hybridation avec un ou plusieurs défauts d'appariement. Ainsi pour un dimère donné, il n'existe pas moins de 24 configurations différentes d'hybridation. Le second terme ne diffencie donc aucunement les différents types défauts d'appariement.

Ce type de modèle pose un certain nombre de problèmes. Tout d'abord, il se pose un problème d'identification. On note en effet qu'il convient d'identifier des termes qui sont des produits les uns des autres. Sans poser un certain nombre de contraintes supplémentaires, non décrites dans le document Zhang, il n'est donc pas possible de connaître comment un algorithme d'identification, basé uniquement sur les équations décrites ci-dessus, répartit la contribution du dimère $(b_k, b_{k+1})$ à l'énergie de libération entre le terme $\varepsilon(b_k, b_{k+1})$ et le terme $\omega_k$, ni même comment l'algorithme répartit la valeur d'un quotion entre le numérateur et le dénominateur de celui-ci. Dit autrement, dans le meilleurs des cas, en l'état seul le terme $\omega_k \cdot \varepsilon(b_k, b_{k+1})$ est pertinent. La même remarque s'applique évidemment aux termes $\omega_k^\star \cdot \varepsilon^\star(b_k, b_{k+1})$. En outre, toujours concernant l'identification, on remarque que ces termes interviennent dans des exponentielles qui sont présentes dans des dénominateurs de fraction, ce qui rend l'identification encore plus délicate.

Par ailleurs, quand bien même il existerait un algorithme d'identification capable de différentier de manière pertinente les différents termes, seul les termes $\omega_k$ et $\varepsilon(b_k, b_{k+1})$ sont exploitables. Les termes $\omega_k^\star$ et $\varepsilon^\star(b_k, b_{k+1})$ ne sont quant à eux valables que pour les puces à ADN d'apprentissage. En effet, une valeur particulière pour le terme correspondant $\omega_k^\star \cdot \varepsilon^\star(b_k, b_{k+1})$ est obtenue pour les défauts ayant lieu pour la puce d'apprentissage. Si l'on considère à présent la conception ultérieure d'une nouvelle puce à ADN, consistant à tester informatiquement des sondes candidates sur la base des valeurs particulières des termes obtenues sur les puces d'apprentissage, par essence la nouvelle puce à concevoir est différente de la ou des puces d'apprentissage. Ceci implique que les défauts d'appariement ont une forte probabilité, voire il est certain, d'être différents de ceux existants dans la puce d'apprentissage. La valeur du terme $\omega_k^\star \cdot \varepsilon^\star(b_k, b_{k+1})$ n'est donc pas valable. En effet, les termes $\omega_k^\star$ et $\varepsilon^\star(b_k, b_{k+1})$ effectuent une moyenne des hybridations non spécifiques sans précisément connaître les défauts d'appariement qui ont réellement lieu sur la puce à ADN. Ainsi, les paramètres estimés à partir d'un échantillon ne peuvent être généralisable.

D'autres modèles plus complexes ont été proposés, comme par exemple celui décrit dans le document « An improved physico-chemical model of hybridization on high-density oligonucleotide microarrays » de Naoaki Ono et al., Bioinformatics, vol.24, n°10, 2008, qui s'appuient aussi sur cette même approche pour modéliser les hybridations non spécifiques.

Mais d'une manière générale, les modèles de l'état de la technique posent de forts problèmes d'identification, et considèrent les défauts d'appariement de manière indifférenciée, et donc de manière trop peu pertinente, pour que les valeurs des paramètres correspondant aux défauts d'appariement puissent être réutilisées ultérieurement lors d'une phase de conception de puce à ADN. Il n'existe donc pas à ce jour de modèle de l'affinité d'une sonde avec une cible menant à la conception efficace de puce à ADN.

## DESCRIPTION DE L'INVENTION

Le but de l'invention est de proposer une modélisation précise de l'affinité entre une sonde et une cible qu'il s'agisse d'une hybridation parfaite (sans défaut d'appariement) ou d'une hybridation avec un ou plusieurs défauts d'appariement.

A cet effet, l'invention a pour objet un procédé d'estimation de l'affinité $\phi$ d'un premier brin d'ADN, ou « sonde », à s'hybrider avec un deuxième brin d'ADN, ou « cible », pour former un hybride de longueur $L_{bp}$, le procédé comprenant :

- dans chaque division d'un ensemble de $M$ divisions de l'hybride, compter le nombre de fois où chaque hybride d'un ensemble de $P$ hybrides de brins d'ADN est présent dans la division, lesdits hybrides étant de longueur $k$ inférieure à la longueur $L_{bp}$, ou « $k$-hybrides » ;
- pour chaque combinaison de défauts d'appariement d'un ensemble de $L$ combinaisons de défauts d'appariement dans un hybride de longueur $L_{bp}$, déterminer si ledit couple de défauts d'appariement est présent dans ledit hybride ; et
- calculer l'affinité $\phi$ selon la relation :

$$\phi = \sum_{m=1}^{M} \sum_{p=1}^{P} x_{m,p} \cdot \hat{\beta}_{m,p} + \alpha$$

expression dans laquelle :

- $\forall (m,p) \in [1,M] \times [1,P]$, $\hat{\beta}_{m,p}$ est un scalaire prédéterminé quantifiant la contribution à l'affinité $\phi$ du p[ième] $k$-hybride de l'ensemble de $P$ $k$-hybrides lorsque ce p[ième] $k$-hybride est présent dans la m[ième] zone de ladite division, et $x_{m,p}$ est le nombre de fois où ce p[ième] $k$-hybride est dénombré pour ledit hybride dans la m[ième] zone de ladite division; et
- $\alpha$ est un terme réel.

En d'autres termes, le modèle d'affinité se fonde directement sur la vraie composition des hybrides sonde/cible et prend en compte directement l'influence de sous-hybrides contenus dans l'hybride. De cette manière, chaque défaut d'appariement entre la sonde et la cible est explicitement et individuellement pris en compte. Ceci permet notamment de déterminer l'affinité d'une sonde avec sa cible spécifique, mais également l'affinité de la sonde avec une cible lors d'une réaction croisée, et donc en présence de défauts d'appariement.

En outre, un modèle selon l'invention est linéaire, contrairement aux modèles de l'état de la technique qui considèrent une affinité qui est proportionnelle à une exponentielle. En raison la linéarité du modèle, il est possible de mettre en œuvre des algorithmes d'identification se fondant sur des problèmes convexes. Comme cela est connu en soi, ce type d'identification est le plus robuste, le plus rapide et le plus précis.

L'estimation de l'affinité, mise en œuvre par ordinateur, fait par exemple suite au séquençage de l'un ou des deux brins d'ADN ou le séquençage d'un ARN ou d'un ADN comprenant le brin, séquençage qui produit une séquence numérique de bases azotées dudit brin, séquence mémorisée dans une mémoire informatique. Le comptage des k-hybrides correspond quant à lui à une mesure sur la structure du double brin issu de l'hybridation des premier et second brins, mesure qui est ensuite traitée pour estimer l'affinité. Par exemple, le procédé comporte a) le séquençage du premier brin d'ADN de manière à produire une première séquence numérique de bases azotées constituant ledit brin, b) la détermination d'une seconde séquence numérique de bases azotées constitutives d'un second brin d'ADN (e.g. par séquençage d'un brin connu, par construction ad hoc de la séquence, par détermination du brin strictement complémentaire dans le cadre de l'affinité d'un hybride parfait, etc.), c) le comptage des k-hybrides est alors réalisé sur les première et seconde séquences numériques hybridées.

Le premier brin est par exemple une sonde de puce à ADN et le second brin est une portion d'un transcrit cible de la puce à ADN, ce qui permet notamment de tester la qualité de la puce à ADN en déterminant l'affinité. Dans un autre exemple d'application, le premier brin est un brin d'ARN messager, par exemple d'un virus, et le second brin est un brin ayant pour fonction de s'attacher de manière spécifique et stable avec le premier brin pour en bloquer la transcription (thérapie génique par utilisation d'un brin dit « antisens »). Connaître l'affinité de l'hybride permet ainsi de caractériser l'efficacité de la thérapie.

Selon un mode de réalisation :

$$\alpha = \sum_{l=1}^{L} y_l . \hat{\delta}_l + \pi$$

expression dans laquelle $\forall l \in [1,L]$, $\hat{\delta}_l$ est un scalaire prédéterminé quantifiant la contribution à l'affinité $\phi$ dudit $l^{ième}$ couple de défauts d'appariement, $\tilde{y}_l = 1$ si ledit $l^{ième}$ couple de défauts d'appariement est présent dans ledit hybride et $y_l = 0$ sinon, $\pi$ est un réel, avantageusement égal à 0.

Plus particulièrement, le procédé comprend :

- pour chaque couple d'un ensemble de $N$ couples d'apprentissage comprenant chacun un premier et un second brins d'ADN aptes à former ensemble un hybride de longueur $L_{bp}$, mettre en présence une quantité du premier brin d'ADN dudit couple avec une quantité du second brin d'ADN dudit couple, et mesurer une intensité $I_n$ représentative de la quantité d'hybrides de brins d'ADN formés suite à cette mise en présence, les hybrides desdits couples de calibration comprenant au moins une fois chaque $k$-hybride de l'ensemble de $P$ $k$-hybrides ; et

- le calcul d'un vecteur $\widehat{\mathbf{B}} \in \mathbb{R}^{P.M}$, d'un vecteur $\widehat{\Theta} \in \mathbb{R}^{N}$ et d'un vecteur $\widehat{\Delta} \in \mathbb{R}^{L}$ minimisant une distance D entre un vecteur $I = (I_1 \dots I_n \dots I_N)^T \in \mathbb{R}^N$ des intensités mesurées et un vecteur $M = (M_1 \dots M_n \dots M_N)^T \in \mathbb{R}^N$ de prédiction du vecteur $I$ des intensités mesurées, ledit calcul étant réalisé en résolvant un problème d'optimisation selon les relations :

$$\left(\widehat{B}, \widehat{\Theta}, \widehat{\Delta}\right) = arg \min_{B, \Theta, \Delta} D\left(I, M\right)$$

$$\forall n \in [1, N], M_n = \theta_n . (X_n . B + Y_n . \Delta)$$

expressions dans lesquelles :

- $\Theta = (\theta_1 \dots \theta_n \dots \theta_N)^T$ est un vecteur de $\mathbb{R}^N$, où $\forall n \in [1, N]$, $\theta_n$ est un scalaire codant une quantité de premier et/ou de second brins d'ADN mis en présence pour le $n^{ième}$ couple de calibration;

- $\forall n \in [1,N]$, $X_n = (X_{n,1} \dots X_{n,m} \dots X_{n,M})$ est une matrice ligne de design prédéterminée de $\mathbb{R}^{P.M}$, où $\forall m \in [1,M]$, $X_{n,m} = (x_{n,m,1} \dots x_{n,m,p} \dots x_{n,m,P})$ est une matrice ligne de $\mathbb{R}^P$ et $\forall p \in [1,P]$, $x_{n,m,p}$ est le nombre de fois où le $p^{ième}$ $k$-hybride est présent dans la $m^{ième}$ zone de ladite division pour l'hybride formé des premier et second brins d'ADN du $n^{ième}$ couple de calibration;

- $B = (B_1 \dots B_m \dots B_M)^T$ est un vecteur de $\mathbb{R}^{P.M}$, où $\forall m \in [1,M]$, $B_m = (\beta_{m,1} \dots \beta_{m,p} \dots \beta_{m,P})^T$ est un vecteur de $\mathbb{R}^P$, avec $\forall p \in [1,P]$, $\beta_{m,p}$ est un scalaire quantifiant la contribution à l'affinité d'un hybride de longueur $L_{bp}$ du $p^{ième}$ $k$-hybride de l'ensemble de $P$ $k$-hybrides lorsque ce $p^{ième}$ $k$-hybride est présent dans la $m^{ième}$ zone de ladite division;

- $\forall n \in [1,N]$, $Y_n = (y_{n,1} \dots y_{n,l} \dots y_{n,L})$ est une matrice ligne de design prédéterminée de $\mathbb{R}^L$, où $\forall l \in [1,L]$, $y_{n,l} = 1$ si $l^{ième}$ couple de défauts d'appariement est présent dans l'hybride formé des premier et second brins d'ADN du $n^{ième}$ couple de calibration ; et

- $\Delta = (\delta_1 \dots \delta_l \dots \delta_L)^T$ est un vecteur de $\mathbb{R}^L$, où $\forall l \in [1,L]$, $\delta_l$ est un scalaire quantifiant la contribution à l'affinité d'un hybride de longueur $L_{bp}$ dudit I$^{ième}$ couple de défauts d'appariement.

Selon un mode de réalisation :

- les $k$-hybrides ont une longueur $k$ comprise entre 2 et 7 ; et
- le nombre $M$ de zones de ladite division est compris en 2 et 25-$k$.

Plus particulièrement, le nombre $M$ de zones est compris entre 3 et 15. Les $k$-hybrides ont notamment une longueur $k$ comprise entre 3 et 5.

Selon une variante, la résolution du problème d'optimisation est résolu sous la contrainte supplémentaire selon la relation :

$$\sum_{i=1}^{I} \theta_i^2 = \alpha$$

où $I$ est le nombre d'ARN différents, $\alpha$ est un scalaire positif prédéterminé, avantageusement égal à $I$.

Selon une variante, le problème d'optimisation est résolu de manière itérative :

- en fixant à l'itération $i$ les vecteurs $\mathbf{B}$, $\Delta$ à leurs valeurs calculées à l'itération précédente $i-1$ et en résolvant le problème d'optimisation selon les relations :

$$\left(\widehat{\boldsymbol{\Theta}}(i)\right) = arg \min_{\Theta(i)} D\left(\boldsymbol{I}, \boldsymbol{M}(i)\right)$$

$$\forall n \in [1, N], M_n(i) = \theta_n(i).\left(\boldsymbol{X}_n.\boldsymbol{B}(i-1) + \boldsymbol{Y}_n.\boldsymbol{\Delta}(i-1)\right)$$

- en fixant à l'itération $i+1$ le vecteur $\Theta$ à sa valeur calculée à l'itération $i$, et en résolvant le problème d'optimisation selon les relations :

$$\left(\widehat{\boldsymbol{B}}(i+1), \widehat{\boldsymbol{\Delta}}(i+1)\right) = arg \min_{B(i+1), \Delta(i+1)} D\left(\boldsymbol{I}, \boldsymbol{M}(i+1)\right)$$

$$\forall n \in [1, N], M_n(i+1) = \theta_n(i).\left(\boldsymbol{X}_n.\boldsymbol{B}(i+1) + \boldsymbol{Y}_n.\boldsymbol{\Delta}(i+1)\right)$$

Plus particulièrement, la première itération est réalisée en fixant $\forall n \in [1,N]$, $\boldsymbol{X}_n.\boldsymbol{B}(1) + \boldsymbol{Y}_n.\Delta(1) = 1$

Il est également décrit un procédé d'estimation, mis en œuvre par ordinateur, des contributions $\hat{\beta}_{m,p}$ d'hybrides d'un ensemble de $P$ hybrides de brins d'ADN de longueur $k$, ou « $k$-hybrides », à l'affinité d'un hybride de brins d'ADN de longueur $L_{bp}$, comprenant :

- pour chaque couple d'un ensemble de $N$ couples d'apprentissage comprenant chacun un premier et un second brins d'ADN aptes à former ensemble un hybride de longueur $L_{bp}$, mettre en présence une quantité du premier brin d'ADN dudit couple avec une quantité du second brin d'ADN dudit couple, et mesurer une intensité $I_n$ représentative de la quantité d'hybrides de brins d'ADN formés suite à cette mise en présence, les hybrides desdits couples de calibration comprenant au moins une fois chaque $k$-hybride de l'ensemble de $P$ $k$-hybrides ; et

- le calcul d'un vecteur $\widehat{\mathbf{B}} \in \mathbb{R}^{P.M}$, d'un vecteur $\widehat{\boldsymbol{\Theta}} \in \mathbb{R}^N$ et d'un vecteur $\widehat{\boldsymbol{\Delta}} \in \mathbb{R}^L$ minimisant une distance D entre un vecteur $\boldsymbol{I} = (I_1 \ldots I_n \ldots I_N)^T \in \mathbb{R}^N$ des intensités mesurées et un vecteur $\boldsymbol{M} = (M_1 \ldots M_n \ldots M_N)^T \in \mathbb{R}^N$ de prédiction du vecteur $\boldsymbol{I}$ des intensités mesurées, ledit calcul étant réalisé en résolvant un problème d'optimisation selon les relations :

$$\left(\widehat{\boldsymbol{B}}, \widehat{\boldsymbol{\Theta}}, \widehat{\boldsymbol{\Delta}}\right) = arg \min_{\boldsymbol{B}, \boldsymbol{\Theta}, \boldsymbol{\Delta}} D\left(\boldsymbol{I}, \boldsymbol{M}\right)$$

$$\forall n \in [1, N], \ M_n = \theta_n . (\boldsymbol{X}_n . \boldsymbol{B} + \boldsymbol{Y}_n . \boldsymbol{\Delta})$$

expressions dans lesquelles :

- $\Theta = (\theta_1 \dots \theta_n \dots \theta_N)^T$ est un vecteur de $\mathbb{R}^N$, où $\forall n \in [1, N]$, $\theta_n$ est un scalaire codant une quantité de premier et/ou de second brins d'ADN mis en présence pour le $n^{\text{ème}}$ couple de calibration;

- $\forall n \in [1, N]$, $\boldsymbol{X}_n = (\boldsymbol{X}_{n,1} \dots \boldsymbol{X}_{n,m} \dots \boldsymbol{X}_{n,M})$ est une matrice ligne de design prédéterminée de $\mathbb{R}^{P.M}$, où $\forall m \in [1, M]$, $\boldsymbol{X}_{n,m} = (x_{n,m,1} \dots x_{n,m,p} \dots x_{n,m,P})$ est une matrice ligne de $\mathbb{R}^P$ et $\forall p \in [1, P]$, $x_{n,m,p}$ est le nombre de fois où le $p^{\text{ème}}$ k-hybride est présent dans la $m^{\text{ème}}$ zone de ladite division pour l'hybride formé des premier et second brins d'ADN du $n^{\text{ème}}$ couple de calibration;

- $\boldsymbol{B} = (\boldsymbol{B}_1 \dots \boldsymbol{B}_m \dots \boldsymbol{B}_M)^T$ est un vecteur de $\mathbb{R}^{P.M}$, où $\forall m \in [1, M]$, $\boldsymbol{B}_m = (\beta_{m,1} \dots \beta_{m,p} \dots \beta_{m,P})^T$ est un vecteur de $\mathbb{R}^P$, avec $\forall p \in [1, P]$ $\beta_{m,p}$ est un scalaire quantifiant la contribution à l'affinité d'un hybride de longueur $L_{bp}$ du $p^{\text{ème}}$ k-hybride de l'ensemble de $P$ k-hybrides lorsque ce $p^{\text{ème}}$ k-hybride est présent dans la $m^{\text{ème}}$ zone de ladite division;

- $\forall n \in [1, N]$, $\boldsymbol{Y}_n = (y_{n,1} \dots y_{n,l} \dots y_{n,L})$ est une matrice ligne de design prédéterminée de $\mathbb{R}^L$, où $\forall l \in [1, L]$, $y_{n,l} = 1$ si $l^{\text{ème}}$ couple de défauts d'appariement est présent dans l'hybride formé des premier et second brins d'ADN du $n^{\text{ème}}$ couple de calibration ; et

- $\Delta = (\delta_1 \dots \delta_l \dots \delta_L)^T$ est un vecteur de $\mathbb{R}^L$, où $\forall l \in [1, L]$, $\delta_l$ est un scalaire quantifiant la contribution à l'affinité d'un hybride de longueur $L_{bp}$ dudit $l^{\text{ème}}$ couple de défauts d'appariement.

L'invention a également pour objet un produit programme d'ordinateur enregistré sur support informatique utilisable par un ordinateur comprenant des instructions pour l'exécution d'un procédé selon l'une quelconque des revendications précédentes.

L'invention a également pour objet un procédé de fabrication d'une puce à ADN comprenant des copies d'un brin d'ADN, ou sonde, apte à former un hybride de longueur $L_{bp}$ avec un brin d'acide nucléique cible, notamment d'ADN, de longueur supérieure à $L_{bp}$ sans défaut d'appariement, ledit procédé comportant :

- identifier un ensemble de portions de longueur $L_{bp}$, sur le brin d'ADN cible ;
- pour chaque portion identifiée du brin d'ADN cible, « ou cible candidate » :

  ○ déterminer le brin d'ADN complémentaire, ou « sonde candidate » et calculer une première affinité $\phi$ de la sonde et la cible candidates en mettant en œuvre un procédé d'estimation d'affinité, notamment du type précité ;
  ○ calculer une seconde affinité $\phi$ de la sonde candidate avec chaque élément d'un ensemble de brins nucléiques de référence ne comprenant pas la cible candidate en mettant en œuvre un procédé d'estimation d'affinité, notamment du type précité ;

- sélectionner, parmi les sondes candidates déterminés, au moins une sonde

  ○ la première affinité $\phi$ est supérieure à un premier seuil prédéterminé ; et
  ○ chacune des secondes affinités $\phi$ est inférieure à second seuil strictement inférieur au premier seuil ;

- fabriquer la puce à ADN avec chaque sonde candidate sélectionnée.

En d'autres termes, il a été constaté qu'il est possible de concevoir une puce à ADN en prenant explicitement en compte uniquement l'affinité des sondes. Si uniquement l'affinité est explicitement prise en compte, le procédé selon l'invention prend cependant en compte également, de manière naturelle mais implicite, la spécificité des sondes. En effet, selon le procédé, la sonde retenue selon les critères de seuil décrits ci-dessus est nécessairement spécifique.

Il n'est ainsi pas nécessaire de développer des modèles de spécificité et/ou de mettre en œuvre des tests spécifiques pour évaluer celle-ci. La conception de la puce selon l'invention est donc grandement simplifiée. Ainsi, en utilisant le modèle d'affinité selon l'invention, les premiers tests ont mené à des puces à ADN de qualité au moins égale à celle de l'état de la technique.

## BREVE DESCRIPTION DES FIGURES

L'invention sera mieux comprise à la lecture de la description qui va suivre, donnée uniquement à titre d'exemple, et faite en relation avec les figures annexées dans lesquelles :

- la figure 1 est une vue schématique fonctionnelle d'une unité de traitement d'information mettant en œuvre un procédé de conception de sondes pour une puce à ADN selon l'invention ;
- la figures 2 à 4 sont des schémas illustrant le découpage d'un hybride sonde/cible en k-hybrides ;
- la figure 5 est un schéma illustrant le découpage en trois zones d'un hybride sonde/cible ;
- la figure 6 est un schéma illustrant des couples de défauts d'appariement dans un hybride sonde/cible ;
- les figures 7 à 9 sont des schémas illustrant le choix de sondes candidates pour une puce à ADN ;
- la figure 10 est un organigramme d'un procédé d'apprentissage de contribution de k-hybride à l'affinité d'une sonde avec un transcrit ;
- les figures 11A à 11D sont des graphiques illustrant la variation du coefficient de détermination du modèle d'affinité selon l'invention en fonction de la longueur $k$ des k-hybrides et du nombre $M$ de divisions d'un hybride sonde/cible ;
- la figure 12 est un schéma illustrant un protocole de validation des sondes pour puce à ADN conçues selon le procédé de l'invention ;
- les figures 13A et 13B sont des graphiques illustrant des intensités prédites conformément à l'invention et mesurées sur une puce à ADN ;
- les figures 14A à 14B sont des graphiques illustrant des intensités mesurées en fonction d'intensités prédites respectivement sur deux puces à ADN ;
- la figure 14C est un diagramme en boite sur le coefficient de détermination correspondant aux figures 14A et 14B ;
- les figures 15A à 15G sont des graphiques illustrant la précision des mesures d'une puce à ADN conçue selon le procédé selon l'invention ; et
- les figures 16A à 16C sont des graphiques illustrant la spécificité des sondes d'une puce à ADN conçue selon le procédé de l'invention.

## DESCRIPTION DETAILLEE DE L'INVENTION

A) SYSTEME D'ESTIMATION D'AFFINITE ET DE SELECTION DE SONDES D'UNE PUCE A ADN

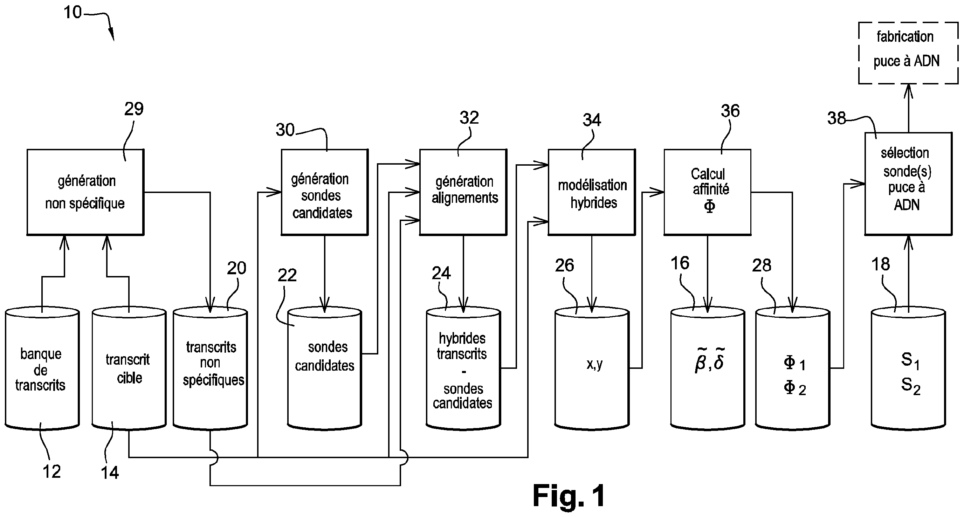

La figure 1 est une vue schématique des blocs fonctionnels mis en œuvre par une unité de traitement d'informations **10**, par exemple un ordinateur personnel, ou tout dispositif à base de microprocesseur, de mémoires RAM, ROM, de masse, etc. apte à implémenter un logiciel comprenant les instructions informatiques nécessaires à la mise en œuvre du procédé, pour la sélection de sondes d'une puce ADN mesurant le niveau d'expression d'un transcrit particulier, ou transcrit « cible ». Cette sélection, dont l'unité **10** met en œuvre un mode de réalisation particulier, vise à identifier une ou plusieurs sondes spécifiques de longueur $L_{bp}$ qui sont spécifiques et affines avec le transcrit cible, et qui ne sont pas, ou faiblement, affines avec d'autres transcrits, ou transcrits « non spécifiques », les sondes sélectionnées étant ensuite utilisées pour la fabrication de la puce à ADN.

A titre d'exemple, la puce à ADN est conçue pour la détection d'un transcrit de rétrovirus endogène présent dans le génome humaine, ou « HERV » pour « *human endogenous retroviruses* », et d'un transcrit de retrotransposon à LTR, ancêtre des rétrovirus infectieux, ou « MalR » pour « *Mammalian-Apparent Long-Terminal Repeat Retrotransposon* ». Les éléments HERV/MarlR représentent jusqu'à 8% du génome humain, soit approximativement 400000 éléments ou loci pouvant chacun produire 0, 1 ou plusieurs transcrits d'une longueur pouvant aller jusqu'à 10000 bases azotées. Par convention, ces éléments sont dénommés sous l'appellation « HERV/MarlR ». On sait que la conception de puce à ADN visant un transcrit HERV/MalR particulier est très difficile en raison de très nombreuses séquences d'ADN dites « répétées » que partagent les éléments HERV/MalR, c'est-à-dire des séquences identiques ou phylogénétiquement très proches les unes des autres présentes en de très nombreux endroits dans le génome humain.

L'unité informatique **10** comporte:

- un premier bloc de mémoire **12** mémorisant une banque numérique de loci HERV/MalR, soit un ensemble de plus 400000 séquences numériques de bases azotées correspondant aux transcrits potentiels HERV/MarlR, e.g. préalablement séquencés d'une manière connue en soi ;

- un deuxième bloc de mémoire **14** mémorisant la séquence numérique codant le transcrit HERV/MarlR cible, par exemple renseignée par le concepteur de la puce à ADN ;
- un troisième bloc de mémoire **16** mémorisant un ensemble $\{\hat{\beta}_{m,p}, \hat{\delta}_l\}$ de coefficient $\hat{\beta}_{m,p}$ et $\hat{\delta}_l$ quantifiant des contributions à l'affinité de k-hybrides et de couples de défauts d'appariement, comme cela sera expliqué plus en détail par la suite ;
- un quatrième bloc de mémoire **18** mémorisant des seuils $S_1$, $S_2$ paramétrant des règles de sélection des sondes de la puce ADN ; et
- des blocs de mémoire **20**, **22**, **24**, **26**, **28** mémorisant des résultats intermédiaires de la sélection des sondes.

L'unité **10** comporte également des blocs de calcul, par exemple des modules logiciels implémentés sur un ordinateur, notamment :

- un bloc **29** de génération d'un ensemble de séquences numériques de bases azotées codant pour les transcrits non spécifiques. Le bloc **28** crée, à partir de la banque de transcrits **12**, un nouvel ensemble de séquences numériques de bases azotées, en lui ôtant le transcrit cible mémorisé dans le bloc **14**. Ce nouvel ensemble code donc les transcrits non spécifiques pour lequel il est recherché des sondes d'affinité faible, et est mémorisé dans le bloc de mémoire **20**. Il existe de nombreuses manières de générer l'ensemble de transcrits non spécifiques. Par exemple, le bloc **28** peut être omis lorsque l'ensemble de la banque **12** contient uniquement ces transcrits. Afin d'alléger les notations, il est par la suite fait indifféremment référence aux séquences correspondant transcrits ou aux transcrits eux-mêmes ;
- un bloc **30** de génération de sondes candidates pour le transcrit cible. De préférence, le bloc **30** identifie chaque sous-séquence de longueur $L_{BP}$ du transcrit cible à chaque position de celui-ci, puis détermine pour chacune de ces sous-séquences la séquence strictement complémentaire en terme de bases azotées. Ces sous-séquences complémentaires forment les sondes « candidates » pour la puce à ADN et sont mémorisées dans le bloc de mémoire **22**. Par analogie, la portion de transcrit cible correspondant à une sonde candidate est désigné sous l'expression de « cible candidate ». Bien entendu d'autres règles de sélection de sondes candidates peuvent être mises en œuvre. Par exemple, certaines portions du transcrit cible peuvent être ignorées s'il est connu au préalable qu'elles ne peuvent donner des sondes appropriées pour la puce à ADN ;
- un bloc **32** d'alignement formant des hybrides entre chaque sonde candidate du bloc de mémoire **22** et les transcrits non spécifiques du bloc de mémoire **20**. Plus particulièrement, le bloc **32** identifie les hybrides comportant au plus deux défauts d'appariement. Pour ce faire, le bloc **32** identifie les hybrides ayant un nombre maximal de paires de bases appariées, en introduisant au besoin un défaut d'appariement de type gap. Les hybrides ainsi identifiés sont mémorisés dans le bloc de mémoire **26**. La limitation du nombre de défauts permet d'accélérer le procédé selon l'invention et de limiter le nombre de coefficients $\{\hat{\beta}_{m,p}, \hat{\delta}_l\}$ nécessaires au calcul de l'affinité. Les inventeurs ont en effet noté qu'à partir de 3 défauts d'appariement, l'affinité d'une sonde avec un transcrit chute, l'intensité d'une puce à ADN correspondant à l'hybride sonde/transcrit étant d'ailleurs noyé dans le bruit de fond. Cette observation est corroborée par l'étude « Custom human endogenous retroviruses dedicated microarray identifies self-induced HERV-Wfamily elements reactivated in testicular cancer upon methylation control » de Gimenez et al., Nucleic Acids Research, avril 2010, vol. 38(7): 2229-2246. Par exemple, le module **32** met en œuvre le logiciel d'alignement « BWA » décrit dans le document « Fast and accurate long-read alignment with Burrows-Wheeler transform », de Li H. et al, Bioinformatices, vol. 26(5) : 589-595, et téléchargeable à l'adresse http://bio-bwa.sourceforge.net/ ;
- un bloc **34** de modélisation de chaque hybride mémorisé dans le bloc de mémoire **24** et de chaque hybride formée d'une sondes candidate et de son transcrit cible à l'aide de « k-hybrides » et de couple de défauts d'appariement, d'une manière décrite ci-après. Cette modélisation produit un ensemble $\{x_{m,p}, y_l\}$ de variables $x_{m,p}$ et $y_l$ pour chaque hybride, coefficients qui sont mémorisés dans le bloc de mémoire **24** ;
- un bloc de calcul **36** qui calcule, pour chaque ensemble $\{x_{m,p}, y_l\}$ mémorisé dans le bloc de mémoire **26**, une affinité $\phi$ de l'hybride correspondant en fonction des coefficients $\hat{\beta}_{m,p}$ et $\hat{\delta}_l$ mémorisés dans le bloc de mémoire **16**, d'une manière expliquée ci-après. Les affinités calculées $\phi$ sont alors mémorisés dans le bloc de mémoire **28**. On note que pour chaque sonde candidate plusieurs affinités $\phi$ sont calculées, une affinité $\phi_1$ pour le transcrit cible de la sonde et une pluralité d'affinités $\phi_2$ pour les transcrits non spécifiques ; et
- un bloc de sélection **38** qui sélectionne au moins une sonde dont les affinités calculées $\phi$, et mémorisées dans le bloc de mémoire **28**, vérifient les règles de sélection paramétrées par les seuils $S_1$, $S_2$ mémorisés dans le bloc de mémoire **18**, d'une manière décrit ci-après.

## B) ESTIMATION DE L'AFFINITE D'UNE SONDE AVEC UN TRANSCRIT

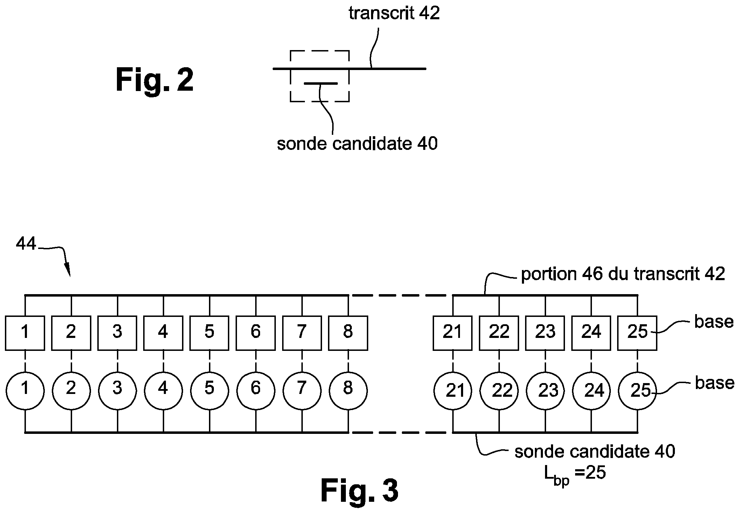

La sélection de sondes pour puce ADN mise en œuvre par l'unité **10** étant en partie définie par la modélisation de l'affinité $\phi$ selon l'invention, cette dernière est tout d'abord détaillée en relation avec les figures 2 à 6. Notamment, pour estimer l'affinité $\phi$ d'une sonde candidate **40** avec un transcrit **42** sur une position particulière de ce dernier, comme illustré à la figure 2, seul l'hybride formé de la sonde **40** et de la portion du transcrit **42** à laquelle la sonde **40** est attachée est considéré. Cet hybride, illustré à la figure 3 sous la référence **44**, comprend donc la sonde **40**, d'une longueur $L_{bp}$ égale par exemple à 25 bases, hybridée avec la portion de transcrit **46** de même longueur. Par convention, les bases de la sonde et de la portion du transcrit sont numérotées de 1 à 25 en partant de la gauche. Selon cette même convention, la position d'une paire de bases appariées dans l'hybride est de même numérotée entre 1 et 25 en partant de la gauche.

Pour l'estimation de l'affinité $\phi$ de la sonde **40** avec la portion de transcrit **46**, l'ensemble des portions k-H$_1$, k-H$_2$, k-H$_3$,...k-H$_{25-k+1}$ de l'hybride de longueur $k$ = 5 bases est identifié, ces portions de longueur k étant désignées sous l'expression de « k-hybrides ». Pour un hybride de longueur L$_{bp}$, un total de $L_{bp}$-k+1 « k-hybrides » est donc identifié. Le modèle de l'affinité $\phi$ selon l'invention calcule l'affinité $\phi$ en fonction de la contribution de chaque k-hybride identifié, la contribution d'un k-hybride dépendant en outre de la position de celui-ci dans l'hybride.

La position d'un k-hybride peut être la position précise dans l'hybride, par exemple déterminée par la position de la paire de bases appariées du k-hybride la plus à gauche dans l'hybride. Ce modèle, dit « toute position », mène donc à considérer $L_{bp}$-k+1 positions différentes. Toutefois, le nombre de positions influence le nombre de paramètres du modèle, et influence donc les ressources en informatiques nécessaires pour le mettre en œuvre, ainsi que la quantité des données d'apprentissage nécessaire.

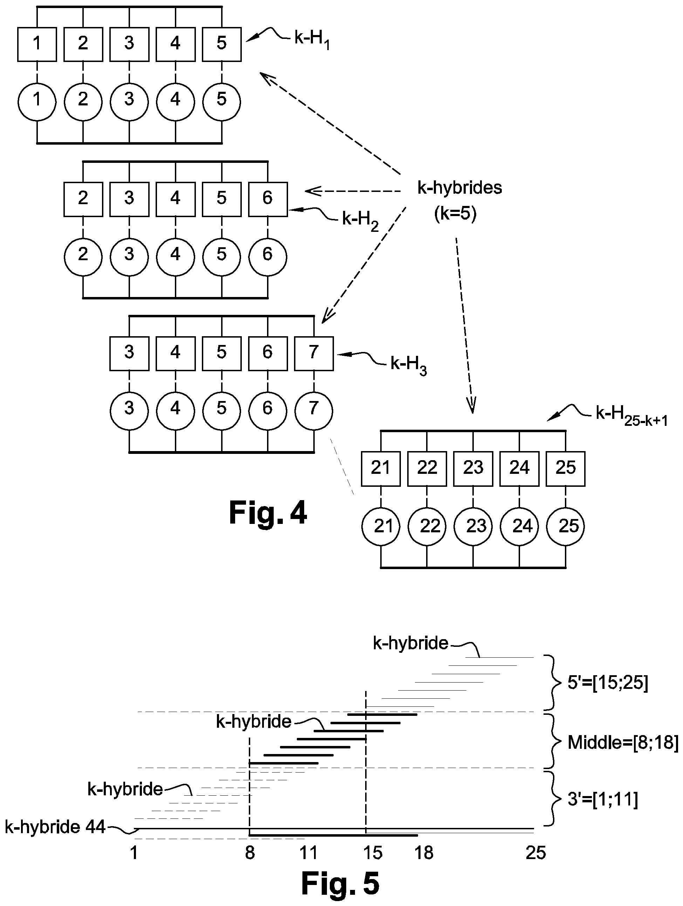

Avantageusement, le nombre de positions d'un k-hybride dans l'hybride est réduit en divisant l'hybride en un nombre limité $M$ de zones. Par exemple, en se référant à la figure 5, l'hybride **44** est divisé en 3 zones dites « 3' », « Middle » et « 5' » de largeur identique. L'ensemble des k-hybrides présents dans l'hybride **44** est illustré par les segments de longueur k= 5, décalés d'une base par rapport aux autres. Selon cette division en trois zones, un k-hybride appartient donc à la première zone « 3' » lorsque sa paire de bases hybridées la plus à gauche est comprise entre les positions 1 et 7 dans l'hybride, à la deuxième zone « Middle » lorsque ladite paire de bases est comprise entre les positions 8 et 14 dans l'hybride, et à la troisième zone « 5' » lorsque ladite paire de bases est comprise entre les positions 15 et 21 dans l'hybride. Un ensemble de k-hybrides identifiés est ainsi obtenu pour chaque zone, à savoir respectivement les ensembles : $\{k - H_1, k - H_2, ..., k - H_7\}_{3'}$, $\{k - H_8, k - H_9, ..., k - H_{14}\}_{Middle}$, $\{k - H_{15}, k - H_{16}, ..., k - H_{21}\}_{5'}$.

La contribution à l'affinité $\phi$ d'un k-hybride dans une zone de l'hybride est par ailleurs calculée préalablement, d'une manière qui sera expliquée plus en détail ci-après, et mémorisée dans les coefficients $\hat{\beta}_{m,p}$ du bloc de mémoire **16**. Plus particulièrement, ayant un alphabet de 5 éléments (A, C, T, G, gap), pour une longueur $k$, il existe $P$ configurations différentes k-H$^1$, k-H$^2$, k-H$^3$,... k-H$^p$, ..., k-H$^p$ pour un k-hybride. Pour chacune de ces configurations k-H$^p$, il est préalablement calculé une contribution $\hat{\beta}_{3',p}$ pour la première zone « 3' », une contribution $\hat{\beta}_{Middle,p}$ pour la deuxième zone « Middle » et une contribution « $\hat{\beta}_{5',p}$ » pour la troisième zone « 5' ».

Une première variante de l'estimation de l'affinité $\phi$ selon l'invention consiste alors :

    a. pour chaque configuration k-H$^p$ de k-hybride, à compter :

- le nombre de fois $x_{3',p}$ où cette configuration apparait dans l'ensemble $\{k - H_1, k - H_2, ..., k - H_7\}_{3'}$ de la première zone « 3' » ;
- le nombre de fois $x_{Middle,p}$ où cette configuration apparait dans l'ensemble $\{k - H_8, k - N_9, ..., k - H_{14}\}_{Middle}$ de la deuxième zone « Middle » ;
- le nombre de fois $x_{5',p}$ cette configuration apparait dans l'ensemble $\{k - H_{15}, k - H_{16}, ..., k - H_{21}\}_{5'}$ de la troisième zone « 5' » ;

    b. puis à calculer l'affinité $\phi$ selon la relation :

$$\phi = \sum_{p=1}^{P} x_{3',p} \cdot \hat{\beta}_{3',p} + \sum_{p=1}^{P} x_{Middle,p} \cdot \hat{\beta}_{Middle,p} + \sum_{p=1}^{P} x_{5',p} \cdot \hat{\beta}_{5',p} \tag{1}$$

Comme on peut le constater, en prenant en compte explicitement la structure d'un hybride, les possibles défauts d'appariement sont donc explicitement pris en compte puisqu'ils interviennent dans les $P$ configurations différentes k-H$^1$, k-H$^2$, k-H$^3$,... k-H$^p$, ..., k-H$^P$.

Pour un nombre quelconque de $M$ zones de l'hybride, y compris un modèle toute position, l'équation précédente se généralise aisément à l'équation :

$$\phi = \sum_{m=1}^{M} \sum_{p=1}^{P} x_{m,p} \cdot \hat{\beta}_{m,p} \tag{2}$$

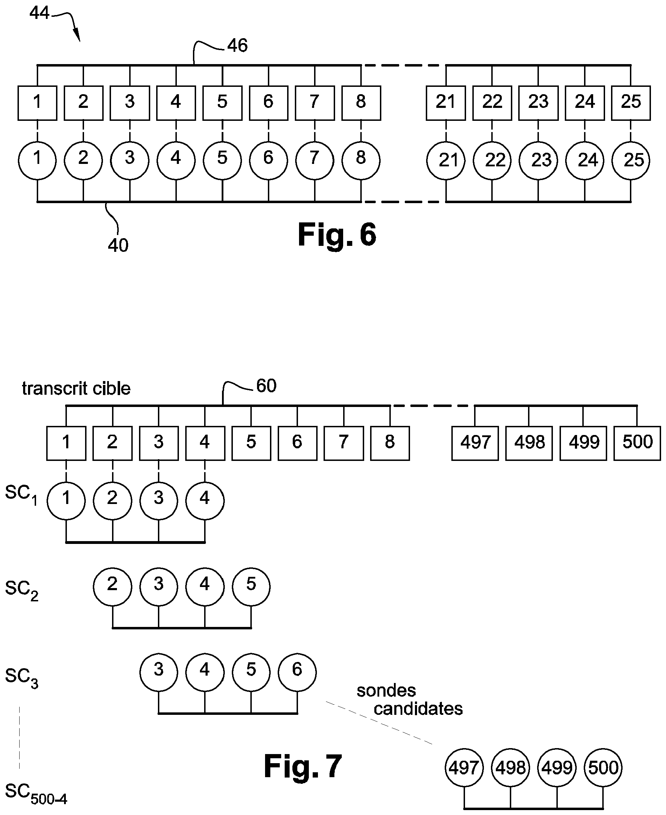

Par ailleurs, il y a un effet de synergie entre les défauts d'appariement présents dans un hybride. Cet effet de synergie, aussi appelé « interaction », est naturellement pris en compte dans les coefficients $\hat{\beta}_{m,p}$ lorsque les défauts d'appariement appartiennent à un même k-hybride. Par contre, lorsque des défauts d'appariement ne sont pas compris ensemble dans un seul k-hybride, et donc sont séparés de plus de $k$ bases, le modèle d'affinité selon la relation (2) ne permet pas d'en tenir compte. Par exemple, en se référant à la figure 6, des défauts d'appariement sont présents aux positions 2, 4, et 7 de l'hybride **44**. Le couple de défauts en positions 2 et 4 et le couple de défauts 4 et 7 étant séparées de moins de $k=5$ bases, l'effet de synergie de ces couple est donc pris en compte par le découpage en k-hybrides de l'hybride. Par contre, les défauts en position 3 et 7 étant séparés de plus de $k= 5$ bases, leur effet de synergie n'est pas pris en compte dans l'estimation de l'affinité de la relation (2).

Avantageusement, le modèle de l'affinité décrit précédemment est complété par un terme prenant en compte l'effet de synergie entre les défauts d'appariement. Plus particulièrement, pour des longueurs $L_{bp}$ et $k$ données, il existe $L$ configurations $C_1$, $C_2$,...,$C_1$...$C_L$ de deux défauts d'appariement séparés de plus de $k$ bases, et pour chacun de ces couples $C_1$, il est préalable calculé une contribution $\hat{\delta}_l$ à l'affinité $\phi$, cette contribution étant mémorisée dans le bloc de mémoire **16**.

Une seconde variante de l'estimation de l'affinité $\phi$ consiste donc en outre à identifier dans l'hybride les défauts d'appariement séparés de plus de $k$ bases et :

c. pour chaque configuration $C_1$ de défauts d'appariement, déterminer si cette configuration est présente dans les couples identifiés. Si tel est le cas, une variable $y_l$ est alors posé égal à 1, et à 0 sinon.
d. puis à calculer l'affinité $\phi$ selon la relation :

$$\phi = \sum_{m=1}^{M} \sum_{p=1}^{P} x_{m,p} \cdot \hat{\beta}_{m,p} + \sum_{l=1}^{L} y_l \cdot \hat{\delta}_l \tag{3}$$

On note ainsi que les défauts et leurs positions précises dans l'hybride sont également pris en compte pour le calcul de l'affinité.

## C) PROCEDE DE SELECTION DE SONDES DE PUCE A ADN

Le procédé de sélection des sondes pour la puce à ADN est à présente décrit plus en détail en relation avec les figures 7 à 9. Ces figures illustrent à titre d'exemple et de clarté des figures, des transcrits spécifiques et non spécifiques de même longueur $L_T$, égale à 500 bases azotées, les bases étant numérotées de 1 à 500. Toujours par soucis de clarté des figures, les sondes candidates sont représentées avec 4 bases seulement, leur longueur réelle $L_{bp}$ étant plus importante, notamment comprise entre 20 et 100 bases azotées.

En se référant à la figure 7, le module **30** de génération des sondes candidates identifie chaque portion de longueur $L_{bp}$ du transcrit cible **60**, les portions étant décalées d'une base les unes par rapport aux autres. Pour chacune de ces portions, le module **30** génère une sonde candidate $SC_1$, $SC_2$,..., $SC_s$,...,$SC_{L_T-L_{bp}+1}$ constituée de la séquence complémentaire de ladite portion. Un total de de $L_T-L_{bp} +1$ sondes candidates est ainsi généré et mémorisé dans le bloc de mémoire **22**.

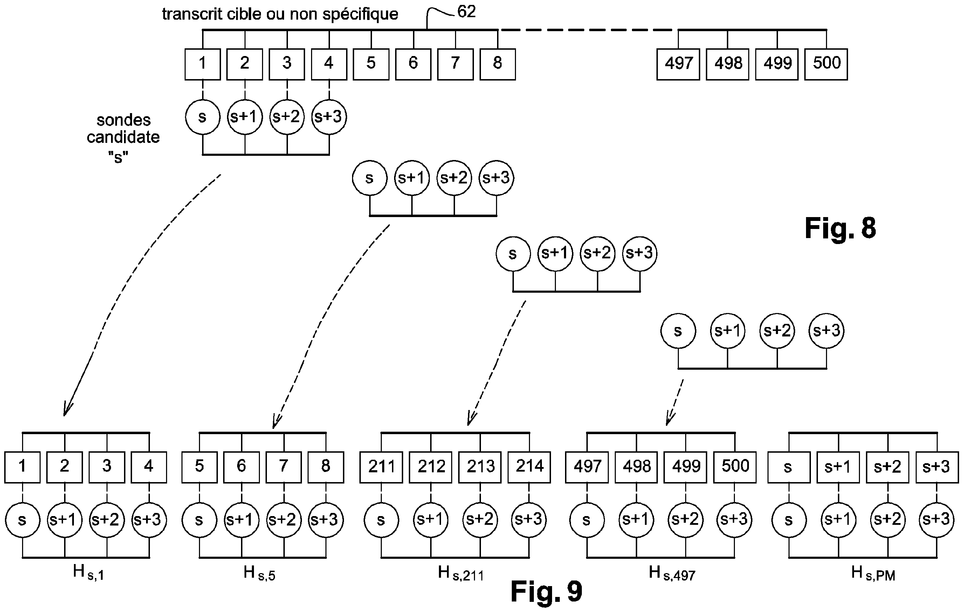

En se référant à la figure 8, le module **32** de génération d'alignements identifie pour chaque couple de transcrit non spécifique **62** et de sonde candidate $SC_s$ **64**, un ensemble d'hybrides comprenant au plus deux défauts d'appariement. Par exemple, sur la figure 7, 4 alignements de ce type sont identifiés, respectivement à la position 1, 5, 211 et 497 du transcrits cibles. Comme illustré à la figure 9, le module **34** de modélisation extrait alors les hybrides $H_{s,1}$, $H_{s,5}$, $H_{s,211}$,$H_{s,497}$, formés de la sonde candidate **64** et des portions du transcrit **62** auxquelles elle est attachées, comme décrit ci-dessus en relation avec les figures 2 et 3, et forme également l'hybride $H_{s,PM}$ constitué de la sonde candidate **64** et de la portion du transcrit spécifique dont elle est issue. Enfin, pour chaque hybride $H_{s,PM}$, $H_{s,1}$, $H_{s,5}$, $H_{s,211}$,$H_{s,497}$ formé, le module **34** identifie les k-hybrides et les couples de défaut d'appariement et détermine pour cet hybride les coefficient $x_{m,p}$ et $y_l$ tel que décrit précédemment, ces paramètres étant mémorisés dans le bloc de mémoire **26**.

Le module **36** calcule alors ensuite les affinités de chaque hybride en fonction des coefficients $x_{m,p}$ et $y_l$ mémorisés dans le bloc de mémoire **24**, des contributions $\hat{\beta}_{m,p}$ de k-hybrides et des contributions $\hat{\delta}_l$ de couple de défauts d'appariement mémorisés dans le bloc de mémoire **16**, ce calcul étant réalisée sur la base de la relation (3). Les affinités ainsi calculées sont alors mémorisées dans le bloc de mémoire **28**. Pour chaque sonde candidate SC_s générée à partir du transcrit cible, il est donc calculé :

- une première affinité $\phi_1$ de la sonde candidate avec son transcrit cible formant avec celui-ci un hybride parfait ;
- des deuxièmes affinités $\phi_2$ de la sonde candidate avec les transcrits non spécifiques, formant avec ceux-ci des hybrides non parfaits ou non.

Enfin, le bloc de sélection **38** sélectionne, en fonction des affinités calculées $\phi_1$ et $\phi_2$ et des paramètres $S_1$ et $S_2$ de sélection mémorisés dans le bloc mémoire **18**, au moins la ou les sondes candidates dont :

    a. la première affinité $\phi_1$ est supérieure à un premier seuil $S_1 > 0$ ;
    b. les deuxième affinité $\phi_2$ sont inférieures à un second seuil $S_2 > 0$, strictement inférieur au premier seuil $S_1$.

En variante, un seul seuil $S_1$ peut être utilisé. La première affinité $\phi_1$ est celle supérieure ou égal au seuil $S_1$ et les deuxièmes affinités $\phi_2$ sont celles qui sont strictement inférieur au seuil $S_1$.
La ou les sondes ainsi sélectionnées sont celles qui sont spécifiques et affines vis-à-vis du transcrit cible. Ces sondes sont alors utilisées pour la fabrication de la puce à ADN qui a pour but de mesurer le niveau d'expression du transcrit cible.
Des règles complémentaires de sélection peuvent également être mises en œuvre. Notamment, en variante, le bloc de sélection **38** sélectionne en ouvre la ou les sondes dont :

    a. la première affinité $\phi_1$ est supérieure au premier seuil $S_1$ ;
    b. au plus N deuxièmes affinité $\phi_2$ sont supérieures au second seuil $S_2$, N étant de préférence égal à 1 ou 2.

Les sondes supplémentaires sélectionnées n'ont pas le caractère spécifique de la première sonde, et peuvent donc s'hybrider de manière stable avec un transcrit non spécifique. Par contre, il existe des puces à ADN dont la construction et l'analyse des mesures permet de distinguer entre une hybridation avec un transcrit cible et une hybridation avec un transcrit non spécifique, ou réaction croisée. De même, une sonde de second rang peut être retenue pour la fabrication de la puce à ADN lorsqu'il est connu que le transcrit cible et le transcrit non spécifique auquel elle s'hybride ont une probabilité faible ou nulle d'être présents ensemble dans l'échantillon biologique objet de la mesure par la puce à ADN. En utilisant également ces sondes dans la puce, la sensibilité de la puce à ADN est donc accrue tout en conservant un caractère spécifique pour cette puce.
Selon l'invention, pour contrôler la spécificité d'une sonde, un score de spécificité *Spec* égal à la différence entre la première affinité $\phi_1$ et la plus grande des deuxièmes affinités $\phi_2$ est calculé pour chaque sonde, c'est-à-dire un score selon la relation :

$$Spec = \phi_1 - max(\phi_2)$$

D) APPRENTISSAGE DES CONTRIBUTIONS $\hat{\beta}_{m,p}$ ET $\hat{\delta}_l$

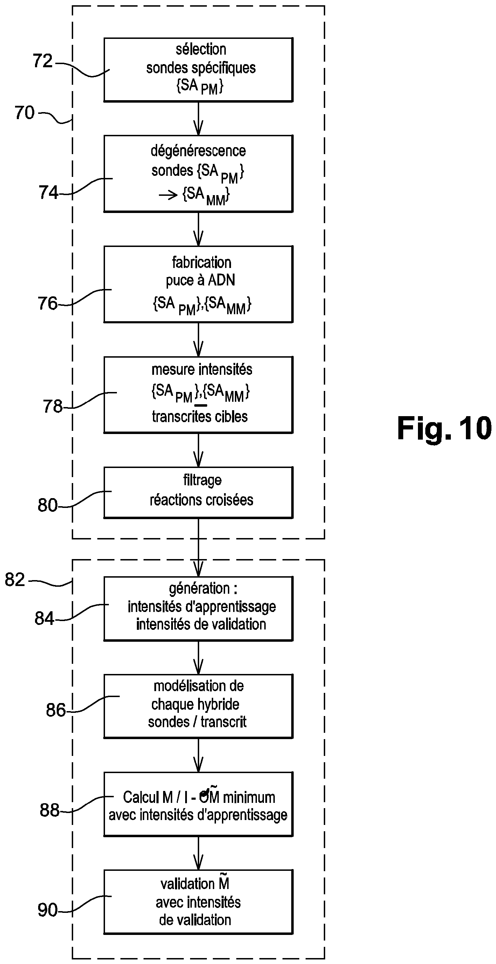

La figure 10 est un organigramme illustrant un procédé d'apprentissage des coefficients $\hat{\beta}_{m,p}$ et $\hat{\delta}_l$ du modèle d'affinité.
Cet apprentissage débute par la construction, en **70**, de données expérimentales d'apprentissage sur la base desquelles identifier les valeurs des coefficients $\hat{\beta}_{m,p}$ et $\hat{\delta}_l$. Plus particulièrement, seule « l'intensité » d'une sonde de puce à ADN, ou d'un dispositif analogue, étant une donnée expérimentale aisément accessible. Les données expérimentales consistent donc en un ensemble $\{I_n\}$ d'intensité de sondes, formant avec des transcrits, des hybrides qui comprennent les k-hybrides et les couples de défauts d'appariement correspondant aux paramètres $\hat{\beta}_{m,p}$ et $\hat{\delta}_l$ recherchés.
Cependant, sans mesure particulière, l'échantillon biologique de départ, objet de la mesure par une puce à ADN, comprend plusieurs transcrits. Chaque hybride stable entre une sonde et un transcrit contribue ainsi à l'intensité de la sonde, sans qu'il soit possible de séparer de manière aisée chaque contribution. La première étape **72** de construction des données expérimentales consiste avantageusement à sélectionner des sondes dont on sait qu'elles sont spécifiques et affines avec les seuls transcrits cibles à partir desquelles elles ont été conçues. Notamment, l'étape **72** consiste à sélectionner un premier ensemble $\{SA_{PM}\}$ de sondes d'apprentissage issues de gènes conventionnels cellulaires (ou « *Protein coding genes* » en anglais). Ces sondes présentent en effet peu ou pas du tout de réaction croisée. Ceci implique tout particulièrement que l'intensité d'une telle sonde correspond substantiellement à l'intensité de la sonde avec son transcrit cible, avec lequel elle forme un hybride parfait.
Dans une étape **74** suivante, un deuxième ensemble $\{SA_{MM}\}$ de sondes d'apprentissage est conçus à partir du premier ensemble {$SA_{PM}$} en modifiant une ou deux bases des sondes de ce dernier. En raison de la très grande spécificité d'une sonde du premier ensemble avec son transcrit cible, les inventeurs ont noté que dégénérer une telle sonde, en changeant une ou deux de ses bases, mène également à une sonde qui est très spécifique avec le transcrit cible. Ainsi, l'intensité d'une sonde dégénérée correspond également substantiellement à l'intensité de l'hybride qu'elle forme avec le transcrit cible, hybride qui présente donc un ou deux défauts d'appariement. Par ailleurs, comme décrit ci-dessous, un filtrage est mis en œuvre pour éliminer d'éventuelles réactions croisées qui pourraient survenir suite à la dégénérescence des sondes du premier ensemble {$SA_{PM}$}. Le premier ensemble {$SA_{PM}$} et le second ensemble {$SA_{MM}$} sont donc sélectionnés pour qu'à eux deux ils comprennent les $P$ configurations possibles de k-hybrides et les $L$ configurations de couples de défaut d'appariement. De préférence, pour la robustesse de l'identification des coefficients $\hat{\beta}_{m,p}$ et $\hat{\delta}_l$, ces ensembles sont choisis pour comprendre plusieurs fois chacune de ces configurations, et de préférence au moins 20 fois.

Une fois les sondes d'apprentissage {$SA_{PM}$} et {$SA_{MM}$} sélectionnées, des puces à ADN sont construites, en **76**, à partir de celles-ci, puis les puces sont utilisées, en **78**, pour mesurer le niveau d'expression des transcrits cibles à partir desquels les sondes {$SA_{PM}$} ont été conçues. Un ensemble {$I$}' d'intensités de sonde est donc obtenu. De manière optionnelle, un filtrage est mis en œuvre, en **80**, pour éliminer les intensités provenant des réactions croisées. Un tel filtrage est par exemple décrit dans le document « Model-based analysis of oligonucleotide arrays : expression index computation and oulier detection », de Li et al, Proceedings of the National Academy of Science, vol. 98(1) :31-36, novembre 2006. Un ensemble {$I$} d'intensités $I$ est alors retenu. Chaque intensité $I$ retenue a donc pour cause unique un seul hybride, à savoir celui formé d'une sonde connue et d'un transcrit connu.

Le procédé d'indentification des coefficients $\hat{\beta}_{m,p}$ et $\hat{\delta}_l$ se poursuit alors par le calcul de ces derniers en fonction des intensités {$I$} lors d'une étape **82**.

Plus particulièrement, en reprenant les notations usuelles dans le domaine des puces à ADN, en raison de la nature des sondes, et éventuellement du filtrage des réactions croisées appliquées, l'intensité $I_{ij}$ d'une sonde « j » est modélisable selon la relation :

$$I_{ij} = \theta_i \times \phi_j \qquad (4)$$

où $\theta_i$ est la quantité d'ARN obtenue par amplification du transcrit i ciblé par la sonde « j » et $\phi_j$ est l'affinité entre la jème sonde et son transcrit cible.

En combinant les relations (3) et (4), l'intensité $I_{ij}$ d'une sonde se réécrit donc formellement :

$$I_{ij} = \theta_i . \left( \sum_{m=1}^{M} \sum_{p=1}^{P} x_{m,p} . \hat{\beta}_{m,p} + \sum_{l=1}^{L} y_l . \hat{\delta}_l \right)_j \qquad (5)$$

où $x_{m,p}$ et $y_l$ correspondent donc ici à la modélisation de l'hybride en k-hybrides et couples de défauts d'appariement de l'hybride associé à l'intensité $I_{ij}$, et $\hat{\beta}_{m,p}$ et $\hat{\delta}_l$ des coefficient à identifier.

En adoptant une écriture matricielle, on montre que la relation (5) se réécrit :

$$I_{ij} = \theta_i . \left( \boldsymbol{X}.\widehat{\boldsymbol{B}} + \boldsymbol{Y}.\widehat{\boldsymbol{\Delta}} \right)_j \qquad (6)$$

expression dans laquelle :

$$\boldsymbol{X}^T = (\boldsymbol{X}_1 \dots \boldsymbol{X}_m \dots \boldsymbol{X}_M)^T \in \mathbb{R}^{P.M} \qquad (7)$$

$$\forall m \in [1, M], \boldsymbol{X}_m^T = \left( x_{m,1} \dots x_{m,p} \dots x_{m,P} \right)^T \in \mathbb{R}^M \qquad (8)$$

$$\widehat{\boldsymbol{B}} = (\boldsymbol{B}_1 \dots \boldsymbol{B}_m \dots \boldsymbol{B}_M)^T \in \mathbb{R}^{P.M} \qquad (9)$$

$$\forall m \in [1, M], \boldsymbol{B}_m = \left( \beta_{m,1} \dots \beta_{m,p} \dots \beta_{m,P} \right)^T \qquad (10)$$

$$Y^T = (y_1 \dots y_l \dots y_L)^T \in \mathbb{R}^L \qquad\qquad (11)$$

$$\widehat{\Delta} = (\delta_1 \dots \delta_l \dots \delta_L)^T \in \mathbb{R}^L \qquad\qquad (12)$$

où $T$ est le symbole de la transposée, la notation « $V \in \mathbb{R}^a$ » désigne un vecteur colonne réel de $\mathbb{R}^a$, et donc un vecteur colonne comportant $a$ composantes réelles.

On note que le terme de droite de la relation (6) est non linéaire puisqu'il est égal à un produit. Par contre, on note que le terme $X.\widehat{B} + Y.\widehat{\Delta}$ est linéaire en les termes $\widehat{B}$ et $\widehat{\Delta}$ et que les matrices $X$ et $Y$ sont connues puisque l'hybride correspondant à l'intensité $I_{ij}$ est connu.

Dans une variante de l'invention, les quantités d'ARN sont contrôlées et connues *a priori*, de sorte que la relation (6) devient linéaire. Le terme $\widehat{\Theta}$ du problème d'optimisation décrit ci-dessous est donc également fixé et connu de sorte que le problème est convexe et est donc solvable plus simplement. Toutefois, le contrôle de la quantité d'ARN est une technique complexe et coûteuse. Selon une variante décrite ci-après, une technique classique de mesure par puce à ADN est mise en œuvre, technique qui ne permet pas de connaître *a priori* les quantités d'ARN. Ces quantités sont donc également identifiées.

Pour mémoire, dans les puces à ADN conventionnelles, un transcrit est ciblé par plusieurs sondes, chacune formant avec le transcrit un hybride parfait. En outre, des réactions croisées peuvent également avoir lieu. Ceci explique que les transcrits et les sondes ne soient pas usuellement référencés avec les mêmes indices, comme cela est décrit dans les relations (4)-(6). Toutefois, en raison de la nature des sondes d'apprentissage et du filtrage des réactions croisées, l'intensité se résume, ou est supposée tel, à l'hybride formé d'une sonde et de son transcrit cible de sorte que la notation peuvent être réduite sans risque de confusion à un seul indice « $n$ », notation qui sera ci-après employée afin d'alléger les relations.

Comme cela est classique dans le domaine de l'identification, l'étape de calcul **82** comporte, en **84**, la séparation en deux ensembles de l'ensemble d'intensités $\{I\}$, à savoir en un premier ensemble d'apprentissage $\{I_n\}$ et en un second ensemble de validation $\{I_q\}$. La manière de découper les données expérimentales, la taille de chacun de ces ensembles et les méthodes de validation sont connues en soi et ne seront donc pas détaillées. Par exemple, l'ensemble $\{I_n\}$ comprend 2/3 de l'ensemble $\{I\}$ et l'ensemble $\{I_q\}$ l'autre 1/3, ou bien la validation est mise en œuvre selon la technique du « 10-fold cross-validation ». Il sera supposé que l'ensemble d'apprentissage $\{I_n\}$ comporte $N$ intensités, indexées par convention par l'entier $n \in [1,N]$. Selon la même convention, l'ensemble $\{SA_n\}$ des sondes d'apprentissage et l'ensemble des quantités d'ARN $\{\theta_n\}$ associés à l'ensemble d'apprentissage $\{I_n\}$ est pareillement indexé par l'entier $n$.

L'étape de calcul **82** comprend également une étape **86** de modélisation de chacun des hybrides associés aux intensités $I$ retenues, la modélisation étant identique à celle décrite en relation avec les figures 3 à 6. Pour chaque intensité $I$ de l'ensemble $\{I\}$, il est donc obtenu une matrice ligne $X \in \mathbb{R}^{P.M}$ et une matrice ligne $Y$ de $\mathbb{R}^L$ telle que décrites aux relation (7)-(12). Notamment, pour chaque intensité $I_n$ de l'ensemble d'apprentissage $\{I_n\}$, il est obtenu une matrice $X_n$ et une matrice $Y_n$.

Dans une étape suivante **88**, un algorithme d'identification est mis en œuvre pour minimiser une distance $D$ entre le vecteur des intensités d'apprentissage $I = (I_1 \dots I_n \dots I_N)^T \in \mathbb{R}^N$ et les intensités prédites par le modèle $M = (M_1 \dots M_n \dots M_N)^T \in \mathbb{R}^N$, à savoir la résolution du problème d'optimisation :

$$\left(\widehat{B}, \widehat{\Theta}, \widehat{\Delta}\right) = arg \min_{B,\Theta,\Delta} D\left(I, M\right) \qquad\qquad (14)$$

$$\forall n \in [1,N], \; M_n = \theta_n.\left(X_n.B + Y_n.\Delta\right) \qquad\qquad (15)$$

Le problème d'optimisation des relations (14)-(15) est classique. Toute distance $D$, également appelée « fonction de coût », convient, par exemple la norme euclidienne. De même tout type d'estimateur convient, par exemple un estimateur par régression non linéaire. Comme on peut le noter, le problème des relations (14)-(15) est non convexe et comprend donc plusieurs solutions. Dans une variante, l'algorithme en cherche plusieurs, celle finalement retenue étant par exemple celle présentant l'erreur d'estimation la plus faible lors de la validation avec l'ensemble de validation $\{I_q\}$ ou celle minimisant un critère de type AIC (« *Akaike Information Criterion* ») ou BIC (« *Baysian Information Criterion* »).

Dans une variante privilégiée, l'espace de recherche est restreint en rajoutant la contrainte :

$$\sum_{i=1}^{I} \theta_i^2 = \alpha \qquad (16)$$

où $I$ est le nombre d'ARN différents déposés sur la puce, avec $\alpha$ par exemple égal à $I$.

Les inventeurs ont noté que le problème d'optimisation des relations (14), (15) et (16) présente une seule solution et à la vue des tests menés il est probable que cette solution est l'optimal global, ou à tout le moins un optimum local proche de l'optimum global.

Selon une variante privilégiée, une résolution itérative du problème des relations (14), (15) et (16) est mise en œuvre :

- en fixant à l'itération *i* les vecteurs **B**, $\Delta$ à leurs valeurs calculées à l'itération précédente *i-1* et en résolvant le problème d'optimisation selon les relations :

$$\left(\widehat{\boldsymbol{\Theta}}(i)\right) = arg\, \min_{\Theta(i)} D\left(\boldsymbol{I}, \boldsymbol{M}(i)\right) \qquad (17)$$

$$\forall n \in [1, N],\ M_n(i) = \theta_n(i).\left(\boldsymbol{X}_n.\boldsymbol{B}(i-1) + \boldsymbol{Y}_n.\boldsymbol{\Delta}(i-1)\right) \qquad (18)$$

$$\sum_{i=1}^{I} \theta_i^2 = I \qquad (19)$$

- en fixant à l'itération *i+1* le vecteur $\Theta$ à sa valeur calculée à l'itération *i*, et en résolvant le problème d'optimisation selon les relations :

$$\left(\widehat{\boldsymbol{B}}(i+1), \widehat{\boldsymbol{\Delta}}(i+1)\right) = arg\, \min_{\boldsymbol{B}(i+1), \Delta(i+1)} D\left(\boldsymbol{I}, \boldsymbol{M}(i+1)\right) \qquad (20)$$

$$\forall n \in [1, N],\ M_n(i+1) = \theta_n(i).\left(\boldsymbol{X}_n.\boldsymbol{B}(i+1) + \boldsymbol{Y}_n.\boldsymbol{\Delta}(i+1)\right) \qquad (21)$$

Chacun de ces problèmes est convexe et donc aisément résolu. La première itération est par exemple réalisée en fixant l'affinité de chaque sonde à 1, c'est-à-dire $\forall n \in [1,N]$, $\boldsymbol{X}_n.\boldsymbol{B}(1) + \boldsymbol{Y}_n.\Delta(1) = 1$ et donc en calculant une première valeur initiale $\Theta(1)$ du vecteur $\widehat{\Theta}$. En variante, la première itération est réalisée en fixant $\forall o \in [1,O]$, $\theta_i = \frac{1}{I}$, et en calculant des premières valeurs $\boldsymbol{B}(1)$ et $\Delta(1)$ pour les vecteurs $\widehat{\boldsymbol{B}}$ et $\widehat{\Delta}$. La résolution itérative du problème est alors stoppée lorsque la distance D n'évolue plus, ou de manière non significative, comme cela est connu en soi.

De manière avantageuse, le problème d'optimisation des relations (20)-(21) est résolu en mettant en œuvre une optimisation de réduction par LASSO (en anglais « *Lasso shrinkage method* ») qui consiste à rajouter la contrainte selon la relation :

$$\|\boldsymbol{B}\|_1 + \|\boldsymbol{\Delta}\|_1 \leq \lambda \qquad (22)$$

où $\|\cdot\|_1$ est la norme $L_1$ et $\lambda$ est un paramètre déterminé par l'optimisation LASSO par cross-validation, d'une manière connue en soi. Ce biais permet de réduire la variance de l'estimateur.

A l'issue de l'étape **88**, il est donc obtenu un vecteur $\widehat{\boldsymbol{B}}$ et un vecteur $\Delta$, c'est-à-dire des valeurs $\widehat{\beta}_{m,p}$ et $\widehat{\delta}_l$ quantifiant la contribution des k-hybrides et des couples de défaut d'appariement à l'affinité $\phi$.

Le procédé se termine alors, en **90**, par la validation des coefficients calculés afin de juger de la qualité de ceux-ci. Notamment, l'étape **88** de calcul précédente est mise en œuvre sur l'ensemble $\{I_q\}$ des intensités de validation, ce qui permet d'identifier les quantités d'ARN correspondantes $\{\theta_q\}$. Chaque intensité $I_q$ de l'ensemble $\{I_q\}$ est ensuite estimée en utilisant les contributions à l'affinité calculées sur les intensités d'apprentissage $\{I_n\}$. L'intensité $I_q$ est ainsi estimée selon la relation :

$$\hat{I}_q = \theta_q.\left(X_q.\widehat{\boldsymbol{B}} + Y_q.\widehat{\boldsymbol{\Delta}}\right) \qquad\qquad (23)$$

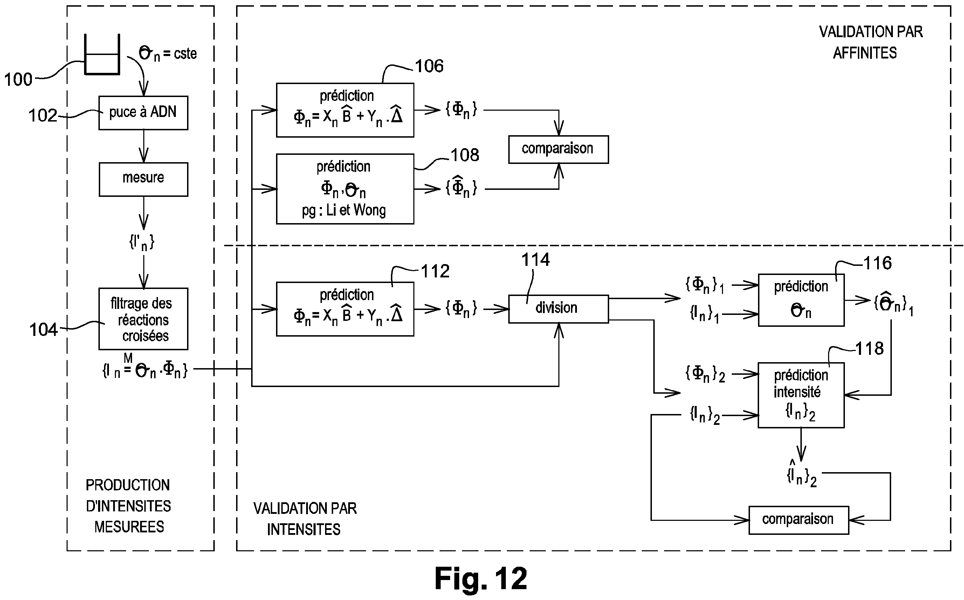

où $\hat{I}_q$ est l'estimation de l'intensité $I_q$, et $X_q$ et $Y_q$ est le modèle de l'hybride associé à l'intensité $I_q$. Une étape de validation par comparaison d'affinité peut également être mise en œuvre, telle que décrit ci-dessous en relation avec la figure 12 détaillée ci-après. L'étape de validation repose notamment sur le fait que la quantité d'ARN d'un transcrit est sensiblement identique d'un puit à un autre, ce qui est le cas en pratique en raison de la nature de la solution déposée sur une puce à ADN. Bien entendu, des mesures particulières peuvent être prises pour garantir cette caractéristique si la solution est produite selon un protocole différent de celui usuellement mis en œuvre pour les puces à ADN, comme par exemple une homogénéisation de la solution.

Des analyses statistiques usuelles sont alors mises en œuvre sur l'erreur d'estimation $I_q$ - $\hat{I}_q$ d'une manière connue en soi.

### E) PARAMETRAGES PRIVILEGIES DE L'AFFINITE ET DE LA SELECTION DES SONDES

D'une manière logique, le modèle d'affinité selon l'invention gagne en précision à mesure que la longueur $k$ et/ou le nombre $M$ de zones augmentent. Toutefois, l'augmentation de ces paramètres pose un certain nombre de problème, au rang desquels la nécessité de ressources informatiques de plus en plus importantes en raison de l'augmentation du nombre de paramètres du modèle, ainsi que la nécessité de concevoir un ensemble de sondes d'apprentissage disposant de plusieurs copies de longs k-hybrides, conception qui est longue et coûteuse.

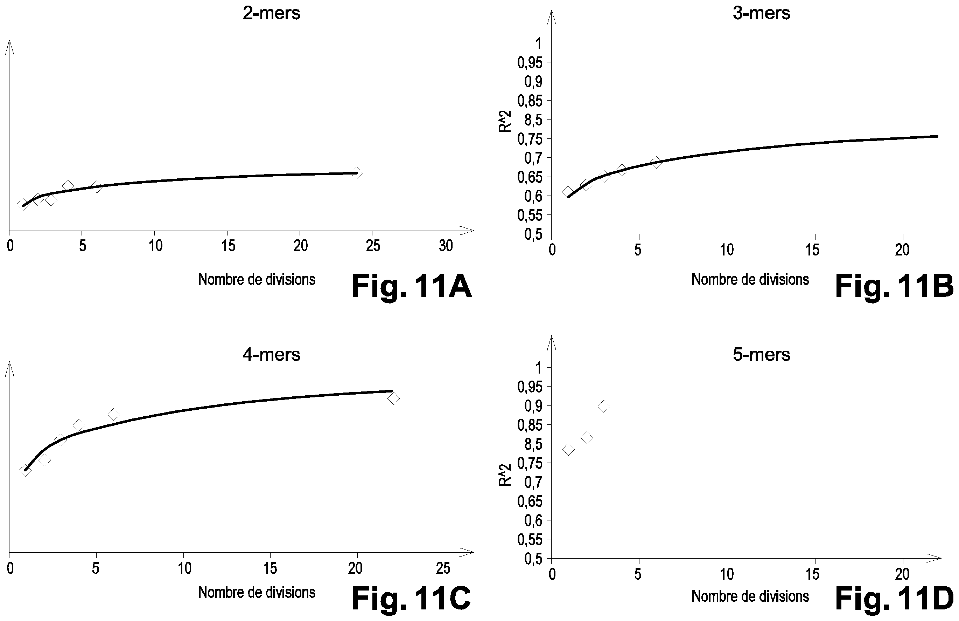

Les inventeurs ont procédé à des tests sur l'influence des paramètres $k$ et $M$ sur la précision du modèle d'affinité. En se référant aux figures 11A à 11D, qui illustrent des courbes du coefficient de détermination $R^2$ du modèle d'affinité en fonction des paramètres $k$ et $M$, les inventeurs ont observé que le gain en précision du modèle progresse très peu une fois dépassé des valeurs pour $k$ et $M$. D'après ces tests, pour des sondes de longueur $L_{bp}$ = 25, les gammes suivantes permettent d'obtenir une bonne précision du modèle, pour un nombre minimal de paramètre et un nombre minimal de sondes d'apprentissage :

- $k$ est compris entre 2 et 7, notamment $k$ compris entre 3 et 5; et
- $M$ est compris en 2 et 25-k, notamment $M$ compris entre 3 et 15

### F) RESULTATS

#### F.1) Matériel et construction des données

Les quatre exemples présentés ci-dessous se fondent sur deux puces à ADN développées par le demandeur. Les sondes ont une longueur égale à 25 bases azotées.

La première puce, dite puce « V2 », comporte un premier compartiment « HERV» développé pour mesurer le transcriptome HERV. Ce compartiment contient 6 familles multicopies rétrovirales correspondant à un peu moins de 6000 transcrits HERV et est décrite dans le document de Pérot et al. « Microarray-based sketches of the HERV transcriptome landscape», PLoS One, 2012;7(6):e40194, juin 2012.

Dans un deuxième compartiment « gènes », au même format que le précédent, sont introduits 513 probesets provenant de la puce à ADN de la société Affymetrix commercialisée sous la référence « HG_U133_Plus2. La puce HG_U133_Plus2 cible des gènes conventionnels cellulaires et est décrite dans la documentation technique « *Design and Performance of the GeneChip® Human Genome U133 Plus 2.0 and Human Genome U133A 2.0 Array* » accessible sur le site internet de la société Affymetrix.

Un troisième compartiment « jeu d'apprentissage » est quant à lui conçu afin d'appréhender l'influence des défauts d'appariement à l'origine de réactions croisées entre des transcrits HERV d'une même famille. Le jeu d'apprentissage provient de 20 probesets de la puce HG_U133_Plus2, destinés par définition à former des hybrides parfaits avec les transcrits qu'elles ciblent. Pour chaque sonde de ces 20 probsets, 185 sondes dégénérées, dont la séquence varie d'un ou deux défauts d'appariement avec la sonde, et ce à différentes positions, ont été conçues. Le jeu d'apprentissage contient donc un ensemble de 37200 sondes.

La puce V2 est donc un outil pour l'apprentissage de modèles de prédication d'affinité (deuxième compartiment) et un outil de validation des modèles appris sur une puce à ADN connue (premier compartiment).

La deuxième puce à ADN, dite « V3 », est une puce à ADN conçue selon la méthodologie présentée ci-dessus, à savoir sur la base du modèle d'affinité de la relation (3) et le procédé de sélection de sonde décrit en relation avec les figures 6 à 8. Notamment, seules les sondes spécifiques et affines sont retenues pour la conception de cette seconde sonde.

La deuxième puce contient environ 400000 éléments HERV/MalR, organisés en plusieurs dizaines de familles. La puce V3 est constituée de plusieurs compartiments (ensemble de sondes) différant les uns des autres soit par les éléments particuliers du génome humain qu'ils ciblent, soit par la méthode de conception des sondes qu'ils contiennent.

La puce V3 comprend notamment trois compartiments « HERV-MalR », « U133 HTA » et « OPTI » qui correspondent à deux types d'éléments du génome humain et deux méthodes de conception de sondes distincts :

- les compartiments U133_HTA et OPTI ciblent les mêmes 1560 gènes, alors que le compartiment HERV-MalR cible environ 400000 éléments HERVs et MaLRs différents des gènes ciblés par les compartiments U133_HTA et OPTI;
- les sondes des compartiments HERV-MalR et OPTI sont conçus en suivant la méthodologie présentée ci-dessus, alors que les sondes du compartiment U133_HTA proviennent de deux puces à ADN Affymetrix, à savoir la puce « HG_U133_Plus2 » (ci-après « U133 ») et la puce commercialisée sous la référence « HTA » respectivement, et sont donc conçues selon la méthodologie propre à la société Affymetrix. Le compartiment U133_HTA est donc en réalité deux ensembles distincts de sondes provenant de deux puces Affymetrix visant les mêmes 1560 gènes.

Plus particulièrement, pour la conception des compartiments HERV-MalR et OPTI, la longueur k des k-hybrides est choisie égale à 5 et le nombre de zones M est choisi égale à 3. Seules sont retenues les sondes dont la première affinité $\phi_1$ est supérieure ou égal à au seuil $S_1$ et dont les deuxième affinité $\phi_2$ sont strictement inférieures au seuil $S_1$. Le seuil $S_1$ est choisi égal à 4 4.

Le compartiment HERV_MalR de la puce V3, le plus volumineux, constitue donc un mode de réalisation de la présente invention. Les deux autres compartiments de la puce V3 (OPTI et U133_HTA) permettent quant à eux une comparaison de l'invention avec des ensembles de sondes conçus selon des méthodes de l'état de la technique. Chacun de ces compartiments contient donc des sondes formant des hybrides parfaits avec leurs transcrits cibles.

F.2.) Précision et choix du modèle de prédiction de l'affinité

La validation d'un modèle de prédiction de l'affinité selon l'invention repose sur le protocole illustré à la figure 12. Un modèle particulier de prédiction ayant été préalablement déterminée (donc pour une longueur $k$ et une division $M$ particulière et des matrices $\hat{B}$ et $\hat{\Delta}$ correspondantes préalablement apprises), le protocole de validation consiste à comparer des affinités de sondes prédites par le modèle avec des affinités « reconstruite » à partir d'intensité mesurée des sondes (branche « validation par affinités », mise en œuvre au moyen de calculs par ordinateur) et/ou à comparer des intensités de sondes prédites par le modèle à des intensités mesurées pour ces sondes (branche « validation par intensité », mise en œuvre au moyen de calculs par ordinateur). Quelle que soit la branche choisie, le protocole débute par la production d'intensités mesurées (branche « production d'intensités mesurées », pour l'essentiel mise en œuvre au moyen de calculs par ordinateur, sauf en ce qui concerne la production et le dépôt de la solution déposée sur la puce à ADN).

La production des intensités mesurées comporte une étape classique de production d'une solution **100** à partir de transcrits ciblés connus par une puce à ADN **102** dont les sondes sont connues, le dépôt de la solution sur la puce, de lavage et de mesure des intensités $\{I'_n\}$ des sondes de la puce. Usuellement, la solution déposée sur la puce à ADN est homogène de sorte que la quantité d'ARN d'un transcrit est identique pour chacun des puits de la puce. Un filtrage **104** des intensités produites est ensuite mise en œuvre pour éliminer les intensités résultants de, ou supposée comme telles, les réactions croisées ou bien corriger les intensités en fonction des réactions croisées, afin d'obtenir des intensités de sonde $\{I_n\}$ correspondant chacune à l'hybride formé de la sonde avec son transcrit cible, et donc chacune modélisable selon la relation $I_n = \theta_n \times \phi_n$, comme cela est décrit ci-dessus.

La branche « validation par affinités » consiste quant à elle à :

- prédire (en **106**) l'affinité $\phi_n$ de chacune des sondes associées aux intensités $\{I_n\}$ en utilisant un modèle selon l'invention $\phi_n = X_n.\hat{B} + Y_n.\Delta$ ;
- estimer (en **108**) une valeur d'affinité $\hat{\phi}_n$ pour chacune des sondes en fonction des intensités $\{I_n\}$. Ce calcul est celui décrit dans l'article de Li et Wong « Model-based analysis of oligonucleotide arrays : Expression index computation and outlier detection », Proceedings of the National Academy of Sciences, 98(1): 31-36, 2001. Ce calcul consiste notamment à minimiser une fonction de coût dépendante des différences $(I_n - \theta_n \times (\phi_n)$ sous la contrainte

$$\sum \theta_n^2 = N,$$ la solution de ce problème d'optimisation étant les valeurs d'affinité $\hat{\phi}_n$ et des prédictions $\hat{\theta}_n$ des quantités d'ARN $\hat{\theta}_n$ ; et

- à comparer (en **110**) les valeurs $\phi_n$ et $\hat{\phi}_n$.

La branche « validation par intensités » consiste quant à elle à :

- prédire (en **112**) l'affinité $\phi_n$ de chacune des sondes associées aux intensités $\{I_n\}$ en utilisant un modèle selon l'invention $\phi_n = \boldsymbol{X}_n.\hat{\boldsymbol{B}} + \boldsymbol{Y}_n.\Delta$ ;
- à diviser (en **114**) l'ensemble des intensités des sondes $\{I_n\}$ en deux sous-ensembles $\{I_n\}_1$ et $\{I_n\}_2$ à l'intérieur de chaque probeset, et de manière correspondante diviser l'ensemble des affinités prédites $\{\phi_n\}$ en deux sous-ensembles $\{\phi_n\}_1$ et $\{\phi_n\}_2$, à proportion de 2/3 et 1/3 ;
- à prédire (en **116**) les quantités d'ARN $\theta_n$ en fonction des ensembles $\{I_n\}_1$ et $\{\phi_n\}_1$. Notamment, cette prédiction consiste en une régression linéaire entre l'ensemble $\{I_n\}_1$ et l'ensemble $\{\theta_n \times \phi_n\}$ puisque les valeurs de $\phi_n$ sont déjà calculées. Une valeur prédite $\hat{\theta}_n$ est ainsi obtenue pour chaque quantité d'ARN $\theta_n$ versée dans les puits de la puce à ADN ;
- à prédire (en **118**) les intensités du sous-ensemble $\{I_n\}_2$ selon la relation $\hat{I}_n = \hat{\theta}_n \times \phi_n$ ;
- à comparer les intensités du sous-ensemble $\{I_n\}_2$ avec leurs prédictions correspondantes $\{\hat{I}_n\}_2$.

Ainsi, les performances du modèle sont évaluées (i) au niveau affinité, en corrélant les affinités prédites par le modèle avec les affinités estimées par le modèle de Li & Wong (2001) et (ii) au niveau intensité, en corrélant celles prédites par le modèle avec les intensités observées, ces comparaisons étant effectuées sondes à sondes. Dans le premier cas, les corrélations sont calculées à l'intérieur de chaque probeset de la puce à ADN en raison de la contrainte

$$\sum \theta_n^2 = N$$ imposé par le modèle de Li & Wong. Autrement dit, au lieu de corréler les affinités prédites par le modèle avec celles de Li & Wong globalement sur l'ensemble des sondes, le calcul des corrélations se fait sondes à sondes pour chaque probeset.

L'exemple présent a pour but d'illustrer la précision de notre modèle d'affinité selon la relation (3), c'est-à-dire son aptitude à prédire finement l'affinité des sondes. Dans cet exemple, une validation par affinité est mise en œuvre.

Neuf modèles de prédiction de l'affinité selon la relation $\phi = \sum_{m=1}^{M} \sum_{p=1}^{P} x_{m,p}.\hat{\beta}_{m,p} + \sum_{l=1}^{L} y_l.\hat{\delta}_l$ sont testés. Les deux variables évaluées sont la taille des k-hybrides ($k$ variant de 3 à 5) et la prise en compte de l'information spatiale selon trois scénarios différents : une sonde est divisé en 1, 3 et 25-k divisions (le dernier cas est appelé « toute position »). Chaque modèle est donc associé à sa propre structure des matrice **X**, **Y**, $\hat{\boldsymbol{B}}$ et $\hat{\Delta}$ et à ses propres valeurs des matrices $\hat{\boldsymbol{B}}$ et $\Delta$. L'apprentissage des modèles est réalisé de la manière décrit en relation avec les étapes **82** à **88** de la figure 10 (les étapes **70-80** de la figure 10 correspondant à la construction du jeu d'apprentissage de la première puce à ADN) sur le jeu d'apprentissage de la première puce en utilisant une approche classique de « 10-folds cross-validation» afin de se prémunir des risques de sur-apprentissage lors de l'apprentissage des paramètres $\hat{\boldsymbol{B}}$, $\hat{\Theta}$, $\hat{\Delta}$.

Pour la validation des neufs modèles selon les intensités, les sondes utilisées sont celles du probeset « CD59 » du compartiment « gènes » de la puce V2, hybridées avec six lignées cellulaires (RWPE1 et cinq lignées qui en dérivent). Ces lignées cellulaires sont des populations homogènes de cellules provenant d'échantillons humains (cellules épithéliales de prostate) qui ont été transformées pour augmenter leur longévité. Les protocoles d'hybridation (amplification, fragmentation, marquage, hybridation sur la puce) et de traitement bioinformatiques des mesures issues de ce celui-ci, sont décrits dans le document de Pérot et al. « *Microarray-based sketches of the HERV transcriptome landscape*». En particulier, les intensités brutes mesurées sur les puces suivent trois étapes de pré-traitements bioinformatiques usuellement suivies dans ce type d'analyse et détaillées dans le document « Exploration, normalization, and summaries of high density oligonucleotide array probe level data, Biostatistics » (Irizarry et al.. 4(2):249-64. Avril 2003). Ces trois étapes sont la correction du bruit de fond, la normalisation inter-puces, et la summarization qui fournit pour chaque probeset une estimation de la quantité d'ARN hybridée à partir des intensités des sondes qui compose ce probeset. Cette dernière étape est réalisée en considérant que l'intensité de chaque sonde est la somme d'un effet affinité cible-sonde propre à la sonde et d'un effet quantité d'ARN commun à l'ensemble des sondes d'un probeset. Chacun de ces effets est estimé de façon robuste à l'aide de la méthode dite « médian polish » (Cf Irizarry et al. 2003).

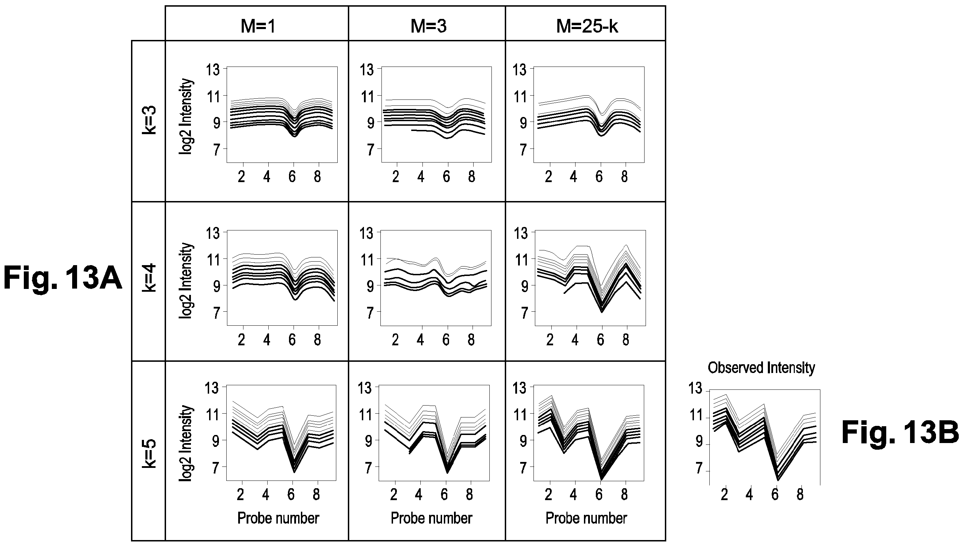

Seize tests en été réalisés, correspondant à une mesure sur le probesets CD59 de 16 puces V2 afin de démonter la précision des modèles même face à une forte variabilité des mesures, notamment en raison de la quantité d'ARN déposée sur les puce V2 qui n'est pas contrôlée de manière précise. Le résultat de ces tests est illustré aux figures 13A et 13B. Sur ces figures, le logarithme de l'intensité est tracé en fonction de la référence des sondes contenues dans le probeset CD59 (au nombre de 9). Chaque tracé comporte en outre l'ensemble des 16 tests réalisés, donc 16 courbes par tracé. Les intensités mesurées sur les 16 probesets CD59 sont illustrés à la figure 13B et les intensités correspondantes prédites en fonction des neuf modèles de prédiction d'affinité sont illustrées à la figure 13A. Dans cette dernière, les neuf modèles sont classés par complexité croissante, de haut en bas par k-hybride croissant et de gauche à droite par complexité croissante de la modélisation spatiale. Deux tendances sont clairement visibles sur la figure 13B: la capacité de ces modèles à modéliser correctement les variations d'affinité à l'intérieur du probeset augmente avec (i) la taille des k-hybrides et (ii) la complexité de l'information spatiale. Nous pouvons noter que le modèle final (5-hybrides 3-divisions) suit très précisément les intensités mesurées sur puce. Ces performances sont illustrées de façon globale dans l'exemple suivant.

F.3.) Validité du modèle de prédiction de l'affinité sur une autre plateforme

L'exemple présent a pour but d'illustrer les performances du modèle de prédiction de l'affinité selon l'invention sur les 513 probesets du compartiment « gènes » de la puce V2 ainsi que de montrer la validité du modèle pour un autre format de puce à ADN, à savoir sur les 3120 probesets du compartiment « U133_HTA » de la puce V3. En effet, alors que les puits de la puce V2, de dimensions égales à ceux de la puce HERV-V2, mesurent $11 \mu m$ de côté, ceux de la puce V3 n'en font que $5 \mu m$.

A cet effet, une validation par affinités et une validation par intensité sont mises en œuvre sur un modèle de prédiction d'affinité caractérisé par une longueur des $k$-hybrides égale à 5 ($k=5$) et par une division des sondes en 3 zones ($M=3$). Comme décrit précédemment, les inventeurs ont noté la bonne performance de ce modèle, et ce même avec une longueur $k$ et un nombre $M$ de zones réduits. Les matrices $\hat{B}$ et $\hat{\Delta}$ de ce modèle sont apprises sur le jeu d'apprentissage de la première puce à ADN.

Les échantillons biologiques utilisés dans cet exemple sont quatre lignées cellulaires différentes du demandeur hybridées simultanément en triplicata sur 12 puces V2 et 12 puces V3 (4 lignées x 3 réplicas = 12 puces). Les protocoles d'hybridation et de traitement bioinformatique utilisés dans cet exemple sont ceux décrits dans l'article Pérot et al. « *Microarray-based sketches of the HERV transcriptome landscape.*».

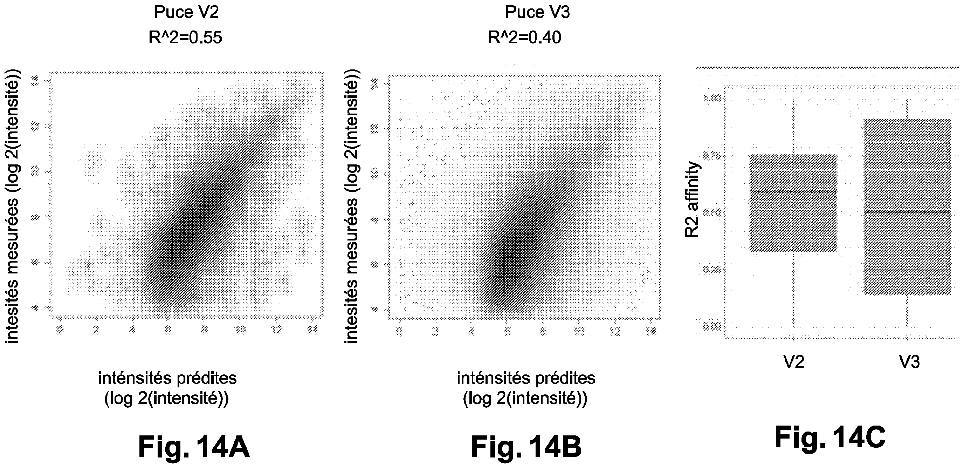

Les figures 14A et 14B illustrent le logarithme des intensités mesurées $\{I_n\}_2$ en fonction du logarithme des intensités prédites $\{\hat{I}_n\}_2$ respectivement pour la puce V2 et la puce V3. La figure 14C illustre la distribution des coefficients de détermination (défini comme le carré du coefficient de détermination, il représente le pourcentage de variation d'une variable expliqué par l'autre) de la puce V2 (compartiment gènes) et de la puce V3 (compartiment U133_HTA).

Les figures 14A et 14B mettent en évidence une bonne cohérence entre les intensités mesurées et les intensités prédites tant que la puce V2 et que sur la puce V3, la corrélation étant légèrement meilleure sur la puce V2 ($R^2 = 0{,}55$) que sur la puce V3 ($R^2 = 0{,}45$). Sur la figure 14C, on observe que la distribution des coefficients de détermination reflètent une bonne concordance entre les affinités prédites par le modèle selon l'invention et celles estimées par le modèle Li & Wong. Ces résultats démontrent donc l'aptitude du modèle à prédire correctement l'affinité des sondes, et ce, quel que soit le format de la puce.

F.4.) Validité du procédé de conception de puce à ADN et précision de la mesure

Une puce à ADN peut être vue comme un instrument de mesure dont le but est de maximiser la variabilité biologique et de minimiser la variabilité technique introduite par l'outil. La variabilité technique, ou erreur, est communément décomposée comme la résultante d'une erreur systématique (ou « biais ») et d'une erreur aléatoire.

Le présent exemple étudie la variabilité technique d'une puce à ADN obtenu selon le procédé de conception selon l'invention. Les résultats présentés dans cet exemple ont pour objectif de montrer que les sondes conçues avec la méthodologie de sélection de sonde selon l'invention, décrite en relation avec les figures 1 à 8, donnent une bonne précision de mesure, et par conséquent, retournent des résultats cohérents avec ceux obtenus avec des puces à ADN commercialisée par la société d'Affymetrix. Les puces d'Affymetrix sont choisies comme référence de comparaison en raison de la qualité qui leur est reconnue. Cette comparaison est menée sur la puce V3 qui comprend des compartiments de sondes conçues selon l'invention (le compartiment « HERV-MaLR » et le compartiment « OPTI ») et un compartiment correspondant à une puce d'Affymetrix (le compartiment « U133_HTA »).

La variabilité technique est étudiée à l'aide de deux critères proposés par le consortium « MicroArray Quality Check » (ou « MAQC ») pour juger de la qualité d'une puce à ADN: la répétabilité (i.e. la variation d'une mesure lorsqu'elle est répétée par un opérateur dans les mêmes conditions. Cette variation reflète l'erreur aléatoire) et la titration monotone (une grandeur proche de la sensibilité d'une puce à ADN permettant de mesurer la cohérence entre les intensités mesurées sur une puce avec les concentrations d'ARN hybridées). Ces critères sont évalués ci-après.

Les échantillons utilisés pour cette évaluation sont ceux utilisés par le consortium MAQC, tels que décrits dans le document « The MicroArray Quality Control (MAQC) project shows inter- and intraplatform reproducibility of gene expression measurements », 24(9):1151-61. Nature Biotechnology, Septembre 2006.

Ces échantillons proviennent de deux échantillons d'ARN du cerveau (A) et d'ARN de référence connus sous l'expression « universal human reference RNA » (B) correspondant à un mélange de 10 lignées cellulaires. Ces deux échantillons sont mélangés dans des proportions 3:1 (C = 0,75xA + 0,25xB) et 1:3 (D = 0,25xA + 0,75xB) pour générer deux échantillons supplémentaires C et D. Chacun de ces échantillons est hybridés en triplicata sur la puce V3. Les protocoles d'hybridation et de traitement bioinformatique utilisés dans cet exemple sont décrits dans l'article Pérot et al. (« *Microarray-based sketches of the HERV transcriptome landscape.*»)

F.4.1.) Etude de la répétabilité

Afin de connaitre la pertinence de la comparaison entre une puce à ADN conçue selon la méthodologie selon l'invention avec une puce d'Affymetrix, une étude est tout d'abord menée pour s'assurer qu'aucun effet confondant ne biaise cette comparaison.

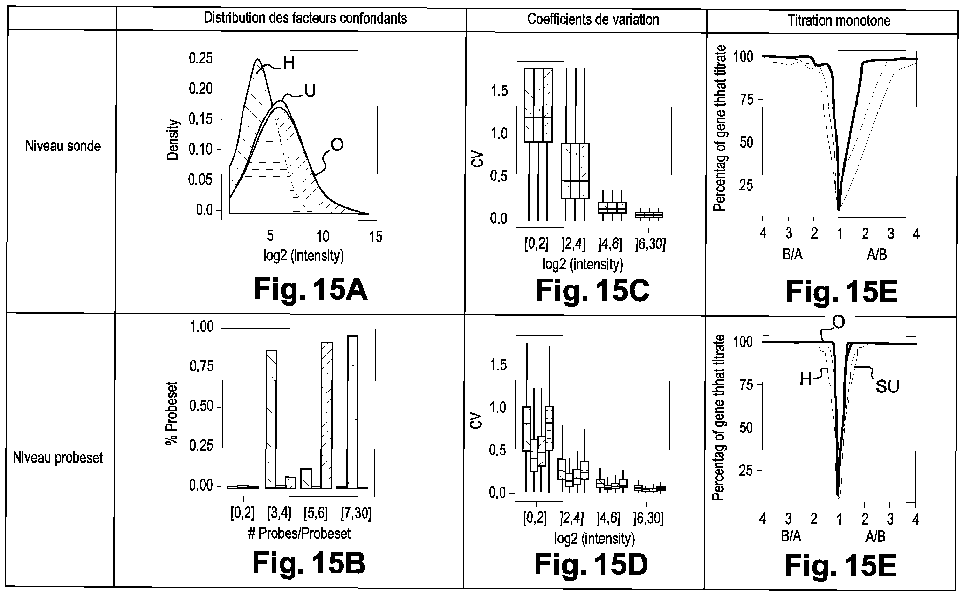

Les résultats de cette étude sont représentés aux figures 15A à 15G qui illustre la précision de la mesure et l'identification de gènes différentiellement exprimés pour chacun des compartiments HERV_MalR (en rose), U133_HTA (en vert) et OPTI (en bleu) de la puce V3.

Les figures 15A et 15B illustrent respectivement les distributions des variables présent un effet confondant, à savoir les intensités et le nombre de sondes par probesets. Sur la figure 15A, la distribution pour le compartiment HERV_MalR est référencée par la lettre « H », la distribution pour le compartiment U133_HTA est référencée par la lettre « U » et la distribution pour le compartiment OPTI est référencée par la lettre « O ». Sur la figure 15B, pour chaque gamme représentée en abscisse, les densités sont, de gauche à droite, celles respectivement des HERV_MalR, U133_HTA et OPTI.

Les figures 15C et 15D illustrent respectivement les coefficients de variations stratifiés par gamme d'intensités au niveau sonde et au niveau probeset. Sur ces figures pour chaque gamme représentée en abscisse, les barres sont, de gauche à droite, celles respectivement des HERV_MalR, U133_HTA et OPTI, et de manière supplémentaire pour la figure 15D celle d'un compartiment « sampled_U133_HTA » décrit ci-après.

A la lecture de ces figures, on constate que les distributions des intensités des échantillons MAQC et celles du nombre de sondes par probesets montrent qu'une grande hétérogénéité existe entre les trois compartiments de la puce V3, nécessitant de stratifier les résultats par intensité et par taille de probeset. La mesure habituellement utilisée pour mesurer la répétabilité est le coefficient de variation entre les réplicas, ce calcul est réalisé au niveau sonde (figure 15C) et au niveau probeset (figure 15D). La comparaison au niveau sonde montre qu'il y a un fort effet de l'intensité sur le coefficient de variation, mais que pour une gamme d'intensité donnée, les trois compartiments présentent des performances identiques. Un même effet de l'intensité est observé au niveau probeset auquel se rajoute un effet taille de probeset : les coefficients de variations sont plus importants dans HERV_MalR que U133_HTA, le premier contenant en majorité trois sondes par probeset alors que le second dépasse sept sondes par probesets dans la plupart des cas. Pour s'assurer que la différence de répétabilité entre ces deux périmètres est bien liée à la taille des probesets et non à la conception des sondes, les probesets U133_HTA sont régénérés en utilisant « X » sondes tirées aléatoirement dans chaque probeset, X étant une variable aléatoire distribuée selon la taille des probesets HERV_MalR. Les probesets nouvellement formés (« sampled_U133_HTA ») présentent des coefficients de variations quasi-identiques à ceux calculés dans le compartiment HERV_MalR. Cela confirme que la répétabilité des sondes étudiées est semblable, et ce, quelle que soit la méthode de conception des sondes considérée.

F.4.2) Titration monotone

Sur les figures 15E et 15F sont représentés les titrations monotones au niveau sonde et probeset. Concernant la figure 15F, le compartiment « sampled_U133_HTA » est référencée par les lettres « SU ». Le critère de performance utilisé dans cet exemple reflète la cohérence entre la concentration d'ARN hybridée sur la puce et le signal observé. En utilisant deux dilutions (C et D) avec des niveaux connus, les rangs des intensités de C et D doivent pouvoir être déduit de l'expression relative des échantillons de départ (A et B). Ainsi, si pour un probeset i nous avons la relation $A_i > B_i$ alors $A_i > C_i > D_i > B_i$. Lorsque l'on représente le pourcentage de probeset respectant cette hiérarchie en fonction de leur ratio A/B et B/A, la forme attendue d'un graphique représentant la titration monotone, tel que représenté aux figures 15E et 15F, est un « V » avec des asymptotes horizontales à l'extrémité des branches. Par conséquent, une méthode de conception des sondes est optimale si le pourcentage de titration atteint le seuil de 100% pour des faibles ratios $A_i/B_i$. Autrement dit, plus les branches du « V » sont rapprochées, meilleure est la méthode associée.

De la même manière que dans l'étude de répétabilité, les trois compartiments sont comparés aux niveaux sondes et probesets, en corrigeant l'effet lié à la taille des probesets dans le second cas. Au niveau sonde, le compartiment OPTI donne de meilleures performances que les deux autres compartiments (figure 15E). Au niveau probeset, les compartiments OPTI et U133_HTA présentent des pourcentages de probesets respectant $A_i > C_i > D_i > B_i$ quasi-identiques et légèrement plus élevés que ceux calculés dans les compartiments HERV_MalR et sampled_U133_HTA (figure 15F). Ainsi, pour l'ensemble des 1560 gènes étudiés, la méthode de conception de puce à ADN selon l'invention donne des résultats légèrement meilleurs qu'Affymetrix au niveau sonde et des résultats équivalents au niveau probeset, et ce, malgré des probesets de plus petite taille.

F.4.3) Gènes différentiellement exprimés

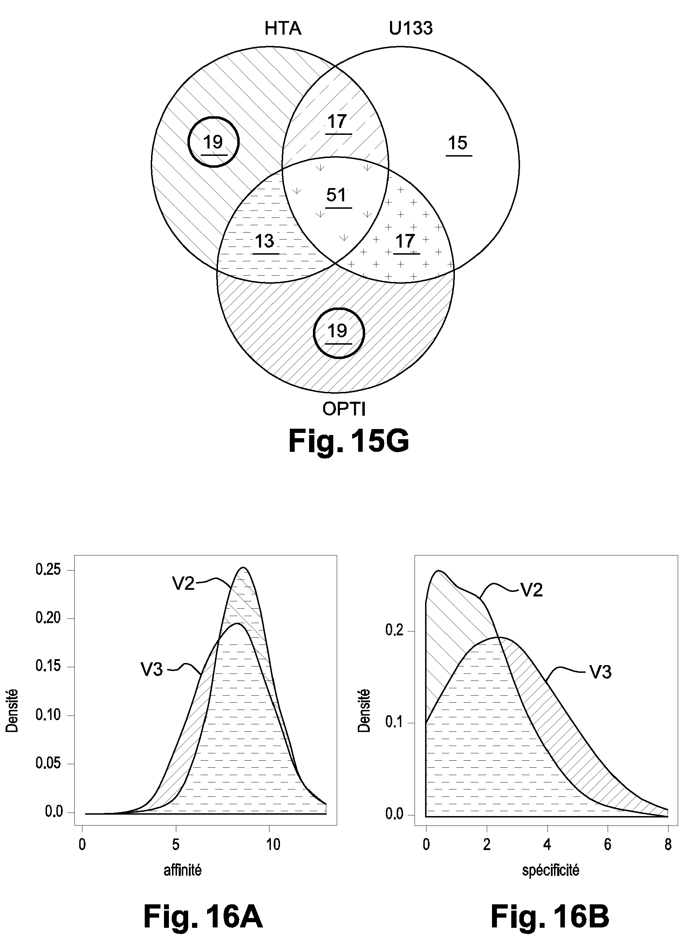

Enfin, la figure 15G présente la cohérence entre les compartiments OPTI, U133 et HTA dans l'identification de gènes différentiellement exprimés entre les échantillons A et B. Ce type d'approche est largement utilisée dans les études utilisant les puces à ADN, en effet ces dernières sont rarement utilisées pour étudier l'expression absolue de transcrits dans un tissu et une condition donnée, mais plutôt dans le cadre d'études visant à identifier des transcrits dont le niveau d'expression varie entre deux ou plusieurs conditions. Sur la figure 15G, le diagramme de Venn représente l'intersection des 100 gènes ayant le plus grand différentiel d'expression dans les compartiments OPTI, U133 et HTA.

Le but de cet exemple est de montrer que les 100 gènes ayant le plus fort différentiel d'expression entre les deux échantillons A et B du MAQC sont comparables dans les compartiments OPTI et U133_HTA (U133 et HTA) de la puce V3. Les gènes différentiellement exprimés sont identifiés à l'aide de la méthode SAM décrite dans le document « Significance analysis of microarrays applied to the ionizing radiation response. »,Tusher VG, Tibshirani R, Chu G. Proceedings of the National Academy of Sciences of the USA. April 2001 24;98(9):5116-21, puis pour chacun des trois compartiments de la puce V3, les 100 gènes avec la plus faible p-value sont retenus. Les intersections entre ces trois compartiments sont représentés sur le diagramme de Venn figure 15G, où l'on peut constater que le nombre de gènes communs entre OPTI et les compartiments Affymetrix (U133 et HTA) est très proches du nombre de gènes partagés entre U133 et HTA. En d'autres termes, la cohérence des résultats entre OPTI et U133/HTA est du même ordre que celle existante entre U133 et HTA, compartiments tous les deux conçus par Affymetrix. Le pourcentage de chevauchement (~65%) est par ailleurs dans la moyenne haute des valeurs trouvées par le consortium MAQC dans leur comparaison inter-plateformes. Ces résultats viennent ainsi démontrer que l'identification de gènes différentiellement exprimés avec les sondes OPTI est cohérente avec les sondes d'Affymetrix.

F.5) Spécificité des mesures

L'exemple présent a pour but de démontrer que le modèle d'hybridation selon l'invention sert non seulement à calculer l'affinité cible-sonde, mais qu'il peut aussi être utilisé pour mesurer la spécificité des sondes. Le compartiment HERV-MaIR de la puce V3 a pour objectif de caractériser de manière spécifique le niveau d'expression des HERVs, organisés en une quarantaine de familles multicopies dans le génome humain. La nature répétée de ces éléments rend leur mesure individuelle difficile.

Pour contrôler la spécificité des sondes, un score de spécificité $Spec = \phi_1 - max(\phi_2)$ est calculé. En d'autres termes, pour une sonde donnée, ce score mesure la différence d'affinité entre l'hybride spécifique et l'hybride non-spécifique la plus stable, i.e. celle qui présente le plus grand risque de réaction croisée. Pour tester la validité de ce score de spécificité, deux types d'expériences peuvent être mise en œuvre :

- pour une sonde donnée, élaborer des ARN « spike-ins » (i.e. synthétisés artificiellement en laboratoire) complémentaires avec des mésappariements et vérifier que la décroissance des intensités est liée à l'augmentation du score de spécificité, ce dernier étant calculé comme la différence d'affinité entre la cible spécifique, absente du mélange réactionnel, et la cible non-spécifique hybridée. Ce type d'approche présente l'avantage de connaitre précisément quels ARNs sont en présence dans le mélange réactionnel ;
- hybrider un même échantillon biologique sur les puces V2 et V3 et corréler les intensités des loci HERV/MarR communs aux deux puces. Plus précisément, le score de spécificité tel que décrit plus haut est calculé pour l'ensemble des sondes, puis lors du calcul de la corrélation, seuls les probesets dont les sondes dépassent un seuil de spécificité donné sont pris en compte. Si le score de spécificité est valide, la corrélation entre les intensités de V2 et V3 devrait augmenter avec le niveau de spécificité. Cette approche plus globale que la précédente est celle choisie dans cet exemple.

Les échantillons biologiques utilisés dans cet exemple proviennent des quatre mêmes lignées cellulaires que celles présentées dans l'exemple F. Les protocoles d'hybridation et de traitement bioinformatique utilisés dans cet exemple sont décrits dans l'article Pérot et al. (« *Microarray-based sketches of the HERV transcriptome landscape* ») et comportent les étapes habituelles d'amplification, de fragmentation, de marquage, d'hybridation sur la puce, suivies des étapes de correction du background, de normalisation et de summarization.

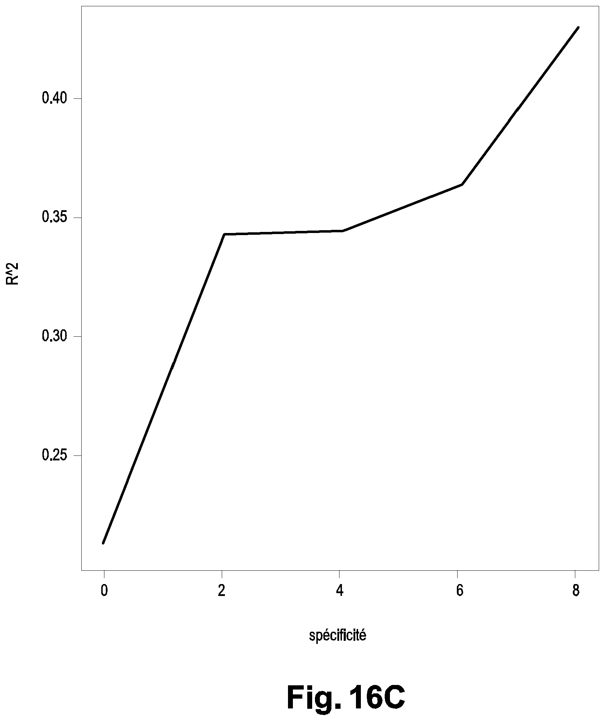

Les figures 16A à 16C illustrent des comparaisons de scores d'affinité et de spécificités des sondes appartenant aux éléments communs des puces V2 et V3. Les figures 16A et 16B illustrent respectivement les distributions en pourcentage des scores d'affinité et de spécificité et la figure 16C illustre le coefficient de détermination entre les intensités mesurées par les puces V2 et V3 en fonction du score de spécificité sur les mêmes éléments communs à ces puces.

Sur les figures 16A et 16B, la comparaison du score d'affinité, c'est-à-dire la première affinité $\phi_1$ et du score de spécificité *Spec* des sondes appartenant aux éléments communs des puces V2 et V3, montre que les sondes V2 sont en moyenne plus affines mais moins spécifiques que celles de la puce V3. On peut ensuite observer, notamment sur la figure 16C, que les mesures effectuées par les puces V2 et V3 sur les mêmes éléments présentent une corrélation croissante à mesure que la spécificité des sondes augmente. Cela signifie que le score de spécificité permet de discerner les sondes peu spécifiques qui cross-hybrident avec des transcrits non-spécifiques des sondes spécifiques qui s'hybrident avec les mêmes transcrits sur les deux puces et donne par conséquent des corrélations plus élevées. Cet exemple démontre que le modèle d'affinité sert non seulement à concevoir des sondes optimales mais aussi à contrôler la spécificité des sondes.

**Revendications**

1. Procédé mis en œuvre par ordinateur d'estimation de l'affinité $\phi$ d'un premier brin d'ADN, ou « sonde », à s'hybrider avec un deuxième brin d'ADN, ou « cible », pour former un hybride de longueur $L_{bp}$, le procédé comprenant :

   - dans chaque division d'un ensemble de $M$ divisions de l'hybride, compter le nombre de fois où chaque hybride d'un ensemble de $P$ hybrides de brins d'ADN est présent dans la division, lesdits hybrides étant de longueur $k$ inférieure à la longueur $L_{bp}$, ou « $k$-hybrides » ;
   - pour chaque combinaison de défauts d'appariement d'un ensemble de $L$ combinaisons de défauts d'appariement dans un hybride de longueur $L_{bp}$, déterminer si ledit couple de défauts d'appariement est présent dans ledit hybride ; et
   - calculer l'affinité $\phi$ selon la relation :

$$\phi = \sum_{m=1}^{M} \sum_{p=1}^{P} x_{m,p} . \hat{\beta}_{m,p} + \alpha$$

   expression dans laquelle :

   - $\forall (m,p) \in [1,M] \times [1,P]$ , $\hat{\beta}_{m,p}$ est un scalaire prédéterminé quantifiant la contribution à l'affinité $\phi$ du $p^{\text{ième}}$ $k$-hybride de l'ensemble de $P$ $k$-hybrides lorsque ce $p^{\text{ième}}$ $k$-hybride est présent dans la $m^{\text{ième}}$ zone de ladite division, et $x_{m,p}$ est le nombre de fois où ce $p^{\text{ième}}$ $k$-hybride est dénombré pour ledit hybride dans la $m^{\text{ième}}$ zone de ladite division; et
   - $\alpha$ est un terme réel.

2. Procédé selon la revendication 1, dans lequel

$$\alpha = \sum_{l=1}^{L} y_l . \hat{\delta}_l + \pi$$

   expression dans laquelle $\forall l \in [1,L]$, $\hat{\delta}_l$ est un scalaire prédéterminé quantifiant la contribution à l'affinité $\phi$ dudit $l^{\text{ième}}$ couple de défauts d'appariement, $y_l$ = 1 si ledit $l^{\text{ième}}$ couple de défauts d'appariement est présent dans ledit hybride et $y_l$ = 0 sinon, $\pi$ est un réel, avantageusement égal à 0.

3. Procédé selon la revendication 2, comprenant :

   - pour chaque couple d'un ensemble de $N$ couples d'apprentissage comprenant chacun un premier et un second brins d'ADN aptes à former ensemble un hybride de longueur $L_{bp}$, mettre en présence une quantité du premier brin d'ADN dudit couple avec une quantité du second brin d'ADN dudit couple, et mesurer une intensité $I_n$ représentative de la quantité d'hybrides de brins d'ADN formés suite à cette mise en présence, les hybrides desdits couples de calibration comprenant au moins une fois chaque $k$-hybride de l'ensemble de $P$ $k$-hybrides ; et
   - le calcul d'un vecteur $\widehat{\mathbf{B}} \in \mathbb{R}^{P.M}$, d'un vecteur $\widehat{\mathbf{\Theta}} \in \mathbb{R}^{N}$ et d'un vecteur $\widehat{\mathbf{\Delta}} \in \mathbb{R}^{L}$ minimisant une distance D entre un vecteur $\boldsymbol{I} = (I_1 \ldots I_n \ldots I_N)^T \in \mathbb{R}^N$ des intensités mesurées et un vecteur $\boldsymbol{M} = (M_1 \ldots M_n \ldots M_N)^T \in \mathbb{R}^N$ de prédiction du vecteur $\boldsymbol{I}$ des intensités mesurées, ledit calcul étant réalisé en résolvant un problème d'optimisation selon les relations :

$$\left(\widehat{B}, \widehat{\Theta}, \widehat{\Delta}\right) = arg \min_{B, \Theta, \Delta} D\left(I, M\right)$$

$$\forall n \in [1, N], \; M_n = \theta_n . (X_n . B + Y_n . \Delta)$$

expressions dans lesquelles :

- $\Theta = (\theta_1 \dots \theta_n \dots \theta_N)^T$ est un vecteur de $\mathbb{R}^N$, où $\forall n \in [1, N]$, $\theta_n$ est un scalaire codant une quantité de premier et/ou de second brins d'ADN mis en présence pour le $n^{\text{ième}}$ couple de calibration;

- $\forall n \in [1, N]$, $X_n = (X_{n,1} \dots X_{n,m} \dots X_{n,M})$ est une matrice ligne de design prédéterminée de $\mathbb{R}^{P.M}$, où $\forall m \in [1, M]$, $X_{n,m} = (x_{n,m,1} \dots x_{n,m,p} \dots x_{n,m,P})$ est une matrice ligne de $\mathbb{R}^P$ et $\forall p \in [1, P]$, $x_{n,m,p}$ est le nombre de fois où le $p^{\text{ième}}$ $k$-hybride est présent dans la $m^{\text{ième}}$ zone de ladite division pour l'hybride formé des premier et second brins d'ADN du $n^{\text{ième}}$ couple de calibration;

- $B = (B_1 \dots B_m \dots B_M)^T$ est un vecteur de $\mathbb{R}^{P.M}$, où $\forall m \in [1, M]$, $B_m = (\beta_{m,1} \dots \beta_{m,p} \dots \beta_{m,P})^T$ est un vecteur de $\mathbb{R}^P$, avec $\forall p \in [1, P]$, $\beta_{m,p}$ est un scalaire quantifiant la contribution à l'affinité d'un hybride de longueur $L_{bp}$ du $p^{\text{ième}}$ $k$-hybride de l'ensemble de $P$ $k$-hybrides lorsque ce $p^{\text{ième}}$ $k$-hybride est présent dans la $m^{\text{ième}}$ zone de ladite division;

- $\forall n \in [1, N]$, $Y_n = (y_{n,1} \dots y_{n,l} \dots y_{n,L})$ est une matrice ligne de design prédéterminée de $\mathbb{R}^L$, où $\forall l \in [1, L]$, $y_{n,l} = 1$ si $l^{\text{ième}}$ couple de défauts d'appariement est présent dans l'hybride formé des premier et second brins d'ADN du $n^{\text{ième}}$ couple de calibration ; et

- $\Delta = (\delta_1 \dots \delta_l \dots \delta_L)^T$ est un vecteur de $\mathbb{R}^L$, où $\forall l \in [1, L]$, $\delta_l$ est un scalaire quantifiant la contribution à l'affinité d'un hybride de longueur $L_{bp}$ dudit $l^{\text{ième}}$ couple de défauts d'appariement.

4. Procédé selon l'une des revendications 1 à 3, *caractérisé* **en ce que** :

- les $k$-hybrides ont une longueur $k$ comprise entre 2 et 7 ; et
- le nombre $M$ de zones de ladite division est compris en 2 et 25-$k$.

5. Procédé selon la revendication 4, *caractérisé* **en ce que** le nombre $M$ de zones est compris entre 3 et 15.

6. Procédé selon la revendication 4 ou 5, *caractérisé* **en ce que** les $k$-hybrides ont une longueur $k$ comprise entre 3 et 5.

7. Procédé selon l'une des revendications 2 à 6, *caractérisé* **en ce que** la résolution du problème d'optimisation est résolu sous la contrainte supplémentaire selon la relation :

$$\sum_{i=1}^{I} \theta_i^2 = \alpha$$

où $I$ est le nombre d'ARN différents, $\alpha$ est un scalaire positif prédéterminé, avantageusement égal à $I$.

8. Procédé selon l'une des revendications 2 à 7, *caractérisé* **en ce que** le problème d'optimisation est résolu de manière itérative :

- en fixant à l'itération $i$ les vecteurs $B$, $\Delta$ à leurs valeurs calculées à l'itération précédente $i-1$ et en résolvant le problème d'optimisation selon les relations :

$$\left(\widehat{\boldsymbol{\Theta}}(i)\right) = arg \min_{\Theta(i)} D\left(\boldsymbol{I}, \boldsymbol{M}(i)\right)$$

$$\forall n \in [1,N], \; M_n(i) = \theta_n(i).\left(\boldsymbol{X}_n.\boldsymbol{B}(i-1) + \boldsymbol{Y}_n.\boldsymbol{\Delta}(i-1)\right)$$

- en fixant à l'itération *i+1* le vecteur $\Theta$ à sa valeur calculée à l'itération *i*, et en résolvant le problème d'optimisation selon les relations :

$$\left(\widehat{\boldsymbol{B}}(i+1), \widehat{\boldsymbol{\Delta}}(i+1)\right) = arg \min_{\boldsymbol{B}(i+1), \boldsymbol{\Delta}(i+1)} D\left(\boldsymbol{I}, \boldsymbol{M}(i+1)\right)$$

$$\forall n \in [1,N], \; M_n(i+1) = \theta_n(i).\left(\boldsymbol{X}_n.\boldsymbol{B}(i+1) + \boldsymbol{Y}_n.\boldsymbol{\Delta}(i+1)\right)$$

**9.** Procédé selon la revendication 8, ***caractérisé* en ce que** la première itération est réalisée en fixant $\forall n \in [1,N]$, $\boldsymbol{X}_n.\boldsymbol{B}(1) + \boldsymbol{Y}_n.\boldsymbol{\Delta}(1) = 1$

**10.** Produit programme d'ordinateur enregistré sur support informatique utilisable par un ordinateur comprenant des instructions pour l'exécution d'un procédé selon l'une quelconque des revendications précédentes.

**11.** Procédé de fabrication d'une puce à ADN comprenant des copies d'un brin d'ADN, ou sonde, apte à former un hybride de longueur $L_{bp}$ avec un brin d'acide nucléique cible, notamment d'ADN, de longueur supérieure à $L_{bp}$ sans défaut d'appariement, ledit procédé consistant à :

- identifier un ensemble de portions de longueur $L_{bp}$ sur le brin d'ADN cible ;
- pour chaque portion identifiée du brin d'ADN cible, « ou cible candidate » :

  ◦ déterminer le brin d'ADN complémentaire, ou « sonde candidate » et calculer une première affinité $\phi$ de la sonde et la cible candidates en mettant en œuvre un procédé selon l'une quelconque des revendications 1 à 9 ;
  ◦ calculer une seconde affinité $\phi$ de la sonde candidate avec chaque éléments d'un ensemble de brins nucléiques ne comprenant pas la cible candidate en mettant en œuvre un procédé selon l'une quelconque des revendications 1 à 9 ;

- sélectionner, parmi les sondes candidates déterminées, au moins une sonde

  ◦ la première affinité $\phi$ est supérieure à un premier seuil $S_1$ prédéterminé ; et
  ◦ chacune des secondes affinités $\phi$ est inférieure à second seuil $S_2$ strictement inférieur au premier seuil $S_1$;

- fabriquer la puce à ADN avec chaque sonde candidate sélectionnée.

**12.** Procédé de fabrication d'une puce à ADN selon la revendication 11, consistant à sélectionner, parmi sondes candidates déterminés, au moins une sonde dont au plus N affinités calculées sont supérieures à un seuil prédéterminé et dont les autres secondes affinités calculés sont inférieure à un second seuil strictement inférieur au premier seuil.

**Patentansprüche**

**1.** Computerimplementiertes Verfahren zur Schätzung der Affinität $\phi$ eines ersten DNA-Strangs, oder "Sonde", mit einem zweiten DNA-Strang, oder "Target", zu hybridisieren, um ein Hybrid der Länge $L_{bp}$ zu bilden, wobei das Verfahren umfasst:

- in jeder Teilung eines Satzes von *M* Teilungen des Hybrids, Zählen der Anzahl von Malen, in der jedes Hybrid aus einem Satz von *P* Hybriden von DNA-Strängen in der Teilung vorliegt, wobei die Hybride, oder "*k*-Hybride", eine Länge *k* aufweisen, die kleiner ist als die Länge $L_{bp}$;

- für jede Kombination von Paarungsdefekten aus einem Satz von $L$ Kombinationen von Paarungsdefekten in einem Hybrid der Länge L$_{bp}$, Bestimmen, ob das Paarungsdefekte-Paar im Hybrid vorliegt; und
- Berechnen der Affinität $\phi$ gemäß der Beziehung:

$$\phi = \sum_{m=1}^{M} \sum_{p=1}^{P} x_{m,p}.\hat{\beta}_{m,p} + \alpha$$

Ausdruck, in dem:

- $\forall (m,p) \in [1,M] \times [1,P]$, $\hat{\beta}_{m,p}$ ein vorbestimmter Skalar ist, der den Beitrag des p-ten $k$-Hybrids aus dem Satz von $P$ $k$-Hybriden an der Affinität $\phi$ quantifiziert, wenn dieses p-te $k$-Hybrid in der m-ten Zone der Teilung vorliegt, und $x_{m,p}$ die Anzahl von Malen ist, in der dieses p-te k-Hybrid bei dem Hybrid in der m-ten Zone der Teilung gezählt wird; und
- $\alpha$ ein reeller Term ist.

2. Verfahren nach Anspruch 1, wobei

$$\alpha = \sum_{l=1}^{L} y_l.\hat{\delta}_l + \pi$$

Ausdruck, in dem $\forall l \in [1,L]$, $\hat{\delta}_l$ ein vorbestimmter Skalar ist, der den Beitrag des l-ten Paarungsdefekte-Paares an der Affinität $\phi$ quantifiziert, $\tilde{y}_l = 1$, wenn das l-te Paarungsdefekte-Paar im Hybrid vorliegt, und andernfalls $y_l = 0$, $\pi$ eine reelle Zahl, vorteilhafterweise gleich 0 ist.

3. Verfahren nach Anspruch 2, umfassend:

- für jedes Paar aus einem Satz von N Anlernpaaren, die jedes einen ersten und einen zweiten DNA-Strang umfassen, die in der Lage sind, zusammen ein Hybrid der Länge $L_{bp}$ zu bilden, Zusammenbringen einer Menge des ersten DNA-Strangs des Paares mit einer Menge des zweiten DNA-Strangs des Paares, und Messen einer Intensität $I_n$, die für die Menge von DNA-Strang-Hybriden repräsentativ ist, welche infolge dieses Zusammenbringens gebildet werden, wobei die Hybride der Kalibrierungspaare jedes $k$-Hybrid aus dem Satz von $P$ $k$-Hybriden mindestens einmal umfassen; und
- das Berechnen eines Vektors $\widehat{\mathbf{B}} \in \mathbb{R}^{P.M}$, eines Vektors $\widehat{\mathbf{\Theta}} \in \mathbb{R}^N$ und eines Vektors $\widehat{\mathbf{\Delta}} \in \mathbb{R}^L$, die eine Distanz D zwischen einem Vektor $\mathbf{I} = (I_1 \dots I_n \dots I_N)^T \in \mathbb{R}^N$ der gemessenen Intensitäten und einem Vektor $\mathbf{M} = (M_1 \dots M_n \dots M_N)^T \in \mathbb{R}^N$ zur Vorhersage des Vektors $\mathbf{I}$ der gemessenen Intensitäten minimieren, wobei die Berechnung unter Lösen eines Optimierungsproblems gemäß den Beziehungen:

$$\left(\widehat{\mathbf{B}}, \widehat{\mathbf{\Theta}}, \widehat{\mathbf{\Delta}}\right) = arg \min_{\mathbf{B},\mathbf{\Theta},\mathbf{\Delta}} D\left(\mathbf{I}, \mathbf{M}\right)$$

$$\forall n \in [1,N], \, M_n = \theta_n.(\mathbf{X}_n.\mathbf{B} + \mathbf{Y}_n.\mathbf{\Delta})$$

ausgeführt wird, Ausdrücke, in denen:

- $\Theta = (\theta_1 \dots \theta_n \dots \theta_N)^T$ ein Vektor von $\mathbb{R}^N$ ist, wobei $\forall n \in [1,N]$, $\theta_n$ ein Skalar ist, der eine Menge des ersten und/oder zweiten DNA-Strangs codiert, die für das n-te Kalibrierungspaar zusammengebracht werden;

- $\forall n \in [1,N]$, $\mathbf{X}_n = (\mathbf{X}_{n,1} \dots \mathbf{X}_{n,m} \dots \mathbf{X}_{n,M})$ eine vorbestimmte Designzeilenmatrix von $\mathbb{R}^{P.M}$ ist, wobei $\forall m \in [1,M]$, $\mathbf{X}_{n,m} = (x_{n,m,1} \dots x_{n,m,p} \dots x_{n,m,P})$ eine Zeilenmatrix von $\mathbb{R}^P$ ist, und $\forall p \in [1,P]$, $x_{n,m,p}$ die Anzahl von Malen ist, in der das p-te k-Hybrid bei dem Hybrid, das vom ersten und zweiten DNA-Strang des n-ten Kalibrierungspaares gebildet wird, in der m-ten Zone der Teilung vorliegt;

- $\boldsymbol{B} = (\boldsymbol{B}_1 \ldots \boldsymbol{B}_m \ldots \boldsymbol{B}_M)^T$ ein Vektor von $\mathbb{R}^{P.M}$ ist, wobei $\forall m \in [1,M]$, $\boldsymbol{B}_m = (\beta_{m,1} \ldots \beta_{m,p} \ldots \beta_{m,P})^T$ ein Vektor von $\mathbb{R}^P$ ist, wobei $\forall p \in [1,P]$, $\beta_{m,p}$ ein Skalar ist, der den Beitrag des p-ten k-Hybrids aus dem Satz von P k-Hybriden an der Affinität eines Hybrids der Länge $L_{bp}$ quantifiziert, wenn dieses p-te k-Hybrid in der m-ten Zone der Teilung vorliegt;

- $\forall n \in [1,N]$, $\boldsymbol{Y}_n = (y_{n,1} \ldots y_{n,l} \ldots y_{n,L})$ eine vorbestimmte Designzeilenmatrix von $\mathbb{R}^L$ ist, wobei $\forall l \in [1,L]$, $y_{n,l} = 1$, wenn das l-te Paarungsdefekte-Paar in dem vom ersten und zweiten DNA-Strang des n-ten Kalibrierungspaares gebildeten Hybrid vorliegt; und

- $\Delta = (\delta_1 \ldots \delta_l \ldots \delta_L)^T$ ein Vektor von $\mathbb{R}^L$ ist, wobei $\forall l \in [1,L]$, $\delta_l$ ein Skalar ist, der den Beitrag des l-ten Paarungsdefekte-Paares an der Affinität eines Hybrids der Länge $L_{bp}$ quantifiziert.

4. Verfahren nach einem der Ansprüche 1 bis 3, **dadurch gekennzeichnet, dass**:

   - die k-Hybride eine Länge k im Bereich zwischen 2 und 7 aufweisen; und
   - die Anzahl M von Zonen der Teilung im Bereich von 2 und 25-k liegt.

5. Verfahren nach Anspruch 4, **dadurch gekennzeichnet, dass** die Anzahl M von Zonen im Bereich zwischen 3 und 15 liegt.

6. Verfahren nach Anspruch 4 oder 5, **dadurch gekennzeichnet, dass** die k-Hybride eine Länge k im Bereich zwischen 3 und 5 aufweisen.

7. Verfahren nach einem der Ansprüche 2 bis 6, **dadurch gekennzeichnet, dass** die Lösung des Optimierungsproblems unter der zusätzlichen Nebenbedingung gemäß der Beziehung:

$$\sum_{i=1}^{l} \theta_i^2 = \alpha$$

gelöst wird, wobei I die Anzahl unterschiedlicher RNAs ist, $\alpha$ ein vorbestimmter positiver Skalar, vorteilhafterweise gleich I ist.

8. Verfahren nach einem der Ansprüche 2 bis 7, **dadurch gekennzeichnet, dass** das Optimierungsproblem iterativ gelöst wird:

   - indem bei der Iteration i die Vektoren **B,** $\Delta$ auf ihre bei der vorhergehenden Iteration i-1 berechneten Werte festgelegt werden, und indem das Optimierungsproblem gemäß den Beziehungen:

$$\left(\widehat{\boldsymbol{\Theta}}(i)\right) = arg \min_{\Theta(i)} D\left(\boldsymbol{I}, \boldsymbol{M}(i)\right)$$

$$\forall n \in [1,N], \; M_n(i) = \theta_n(i).(\boldsymbol{X}_n.\boldsymbol{B}(i-1) + \boldsymbol{Y}_n.\boldsymbol{\Delta}(i-1))$$

gelöst wird,
   - indem bei der Iteration i+1 der Vektor $\Theta$ auf seinen bei der Iteration i berechneten Wert festgelegt wird, und indem das Optimierungsproblem gemäß den Beziehungen:

$$\left(\widehat{\boldsymbol{B}}(i+1), \widehat{\boldsymbol{\Delta}}(i+1)\right) = arg \min_{B(i+1),\Delta(i+1)} D\left(\boldsymbol{I}, \boldsymbol{M}(i+1)\right)$$

$$\forall n \in [1, N], \; M_n(i+1) = \theta_n(i).(X_n.B(i+1) + Y_n.\Delta(i+1))$$

gelöst wird.

**9.** Verfahren nach Anspruch 8, **dadurch gekennzeichnet, dass** die erste Iteration ausgeführt wird, indem $\forall n \in [1, N]$, $X_n.B(1) + Y_n.\Delta(1) = 1$ festgelegt wird.

**10.** Auf einem Datenträger aufgezeichnetes, von einem Computer verwendbares Computerprogrammprodukt, das Anweisungen für die Ausführung eines Verfahrens nach einem der vorstehenden Ansprüche umfasst.

**11.** Verfahren zur Herstellung eines DNA-Chips, der Kopien eines DNA-Strangs, oder einer Sonde, umfasst, welcher in der Lage ist, mit einem Target-Nukleinsäure-, insbesondere DNA-, Strang einer größeren Länge als $L_{bp}$ ein Hybrid der Länge $L_{bp}$ ohne Paarungsdefekt zu bilden, wobei das Verfahren darin besteht:

- am Target-DNA-Strang einen Satz von Abschnitten der Länge $L_{bp}$ zu identifizieren;
- für jeden identifizierten Abschnitt des Target-DNA-Strangs, oder "Kandidaten-Target":

-- den komplementären DNA-Strang, oder "Kandidaten-Sonde", zu bestimmen und eine erste Affinität $\phi$ der Kandidaten-Sonde und des Kandidaten-Targets unter Umsetzung eines Verfahrens nach einem der Ansprüche 1 bis 9 zu berechnen;
-- eine zweite Affinität $\phi$ der Kandidaten-Sonde mit jedem Element aus einem Satz von Nukleinsträngen, der das Kandidaten-Target nicht umfasst, unter Umsetzung eines Verfahrens nach einem der Ansprüche 1 bis 9 zu berechnen;
- unter den bestimmten Kandidaten-Sonden mindestens eine Sonde auszuwählen, bei der
-- die erste Affinität $\phi$ größer ist als eine erste vorbestimmte Schwelle $S_1$; und
-- jede der zweiten Affinitäten $\phi$ kleiner ist als eine zweite Schwelle $S_2$, die streng kleiner ist als die erste Schwelle $S_1$;
- den DNA-Chip mit jeder ausgewählten Kandidaten-Sonde herzustellen.

**12.** Verfahren zur Herstellung eines DNA-Chips nach Anspruch 11, das darin besteht, unter den bestimmten Kandidaten-Sonden mindestens eine Sonde auszuwählen, bei der höchstens N berechnete Affinitäten größer sind als eine vorbestimmte Schwelle, und bei der die anderen zweiten berechneten Affinitäten kleiner sind als eine zweite Schwelle, die streng kleiner ist als die erste Schwelle.

**Claims**

**1.** A method implemented by computer for estimating the affinity $\phi$ of a first DNA strand, or "probe", to be hybridized with a second DNA strand, or "target", to form a hybrid of length $L_{bp}$, the method comprising:

- in each division of a set of $M$ divisions of the hybrid, counting the number of times in which each hybrid of a set of $P$ DNA strand hybrids is present in the division, said hybrids being of length $k$ less than the length $L_{bp}$, or "$k$-hybrids";
- for each combination of mismatches of a set of $L$ combinations of mismatches in a hybrid of length Lbp, determining whether said pair of mismatches is present in said hybrid; and
- calculating the affinity $\phi$ according to the equation:

$$\phi = \sum_{m=1}^{M} \sum_{p=1}^{P} x_{m,p}.\hat{\beta}_{m,p} + \alpha$$

an expression in which:

- $\forall(m,p) \in [1,M] \times [1,P]$, $\hat{\beta}_{m,p}$ is a predetermined scalar quantifying the contribution to the affinity $\phi$ of the $p^{th}$ $k$-hybrid of the set of $P$ $k$-hybrids when this $p^{th}$ $k$-hybrid is present in the $m^{th}$ area of said division, and $x_{m,p}$ is the number of times in which this $p^{th}$ $k$-hybrid is counted for said hybrid in the $m^{th}$ area of said division;

and
- $\alpha$ is a real term.

2.  The method according to claim 1, in which

$$\alpha = \sum_{l=1}^{L} y_l . \hat{\delta}_l + \pi$$

an expression in which $\forall l \in [1,L]$, $\hat{\delta}_l$ is a predetermined scalar quantifying the contribution to the affinity $\phi$ of the 1th pair of mismatches, $\tilde{y}_l = 1$ if the 1th pair of mismatches is present in said hybrid and $y_l = 0$ otherwise, $\pi$ is a real number, advantageously equal to 0.

3.  The method according to claim 2, comprising:

- for each pair of a set of N learning pairs each comprising a first and a second DNA strand capable of together forming a hybrid of length $L_{bp}$, bringing together a quantity of the first DNA strand of said pair with a quantity of the second DNA strand of said pair, and measuring an intensity $I_n$ representative of the quantity of DNA strand hybrids formed following this bringing together, the hybrids of said calibration pairs comprising at least one time each k-hybrid of the set of P k-hybrids; and

- calculating a vector $\widehat{B} \in \mathbb{R}^{P.M}$, a vector $\widehat{\Theta} \in \mathbb{R}^N$ and a vector $\widehat{\Delta} \in \mathbb{R}^L$ minimizing a distance D between a vector $I = (I_1 \dots I_n \dots I_N)^T \in \mathbb{R}^N$ of the measured intensities and a vector $M = (M_1 \dots M_n \dots M_N)^T \in \mathbb{R}^N$ of prediction of the vector I of the measured intensities, said calculation being performed by solving an optimization problem according to the equations:

$$\left(\widehat{B}, \widehat{\Theta}, \widehat{\Delta}\right) = arg \min_{B, \Theta, \Delta} D\left(I, M\right)$$

$$\forall n \in [1, N], M_n = \theta_n . (X_n . B + Y_n . \Delta)$$

expressions in which:

- $\Theta = (\theta_1 \dots \theta_n \dots \theta_N)^T$ is a vector of $\mathbb{R}^N$, in which $\forall n \in [1,N]$, $\theta_n$ is a scalar coding a quantity of first and/or second DNA strands brought together for the nth calibration pair;

- $\forall n \in [1,N]$, $X_n = (X_{n,1} \dots X_{n,m} \dots X_{n,M})$ is a row matrix of predetermined design of $\mathbb{R}^{P.M}$, in which $\forall m \in [1,M]$, $X_{n,m} = (x_{n,m,1} \dots x_{n,m,p} \dots x_{n,m,P})$ is a row matrix of $\mathbb{R}^P$ and $\forall p \in [1,P]$, $x_{n,m,p}$ is the number of times in which the pth k-hybrid is present in the mth area of said division for the hybrid formed by the first and second DNA strands of the nth calibration pair;

- $B = (B_1 \dots B_m \dots B_M)^T$ is a vector of $\mathbb{R}^{P.M}$, in which $\forall m \in [1,M]$, $B_m = (\beta_{m,1} \dots \beta_{m,p} \dots \beta_{m,P})^T$ is a vector of $\mathbb{R}^P$, with $\forall p \in [1,P]$, $\beta_{m,p}$ is a scalar quantifying the contribution to the affinity of a hybrid of length $L_{bp}$ of the pth k-hybrid of the set of P k-hybrids when this pth k-hybrid is present in the mth area of said division;

- $\forall n \in [1,N]$, $Y_n = (y_{n,1} \dots y_{n,l} \dots y_{n,L})$ is a row matrix of predetermined design of $\mathbb{R}^L$, in which $\forall l \in [1,L]$, $y_{n,l} = 1$ if the 1th pair of mismatches is present in the hybrid formed by the first and second DNA strands of the nth calibration pair; and

- $\Delta = (\delta_1 \dots \delta_l \dots \delta_L)^T$ is a vector of $\mathbb{R}^L$, in which $\forall l \in [1,L]$, $\delta_l$ is a scalar quantifying the contribution to the affinity of a hybrid of length $L_{bp}$ of said 1th pair of mismatches.

4. The method according to any of claims 1 to 3, **characterized in that**:

   - the *k*-hybrids have a length *k* of between 2 and 7; and
   - the number *M* of areas of said division is between 2 and 25-*k*.

5. The method according to claim 4, **characterized in that** the number *M* of areas is between 3 and 15.

6. The method according to claim 4 or 5, **characterized in that** the *k*-hybrids have a length *k* of between 3 and 5.

7. The method according to any of claims 2 to 6, **characterized in that** the solving of the optimization problem is solved subject to the additional constraint according to the equation:

$$\sum_{i=1}^{I} \theta_i^2 = \alpha$$

in which *I* is the number of different RNAs, $\alpha$ is a predetermined positive scalar, advantageously equal to *I*.

8. The method according to any of claims 2 to 7, **characterized in that** the optimization problem is solved iteratively:

   - by setting, on the iteration i, the vectors **B**, $\Delta$ to their values calculated on the preceding iteration *i-1* and by solving the optimization problem according to the equations:

$$\left(\widehat{\boldsymbol{\Theta}}(i)\right) = arg \min_{\Theta(i)} D\left(\boldsymbol{I}, \boldsymbol{M}(i)\right)$$

$$\forall n \in [1,N],\ M_n(i) = \theta_n(i).\left(\boldsymbol{X}_n.\boldsymbol{B}(i-1) + \boldsymbol{Y}_n.\boldsymbol{\Delta}(i-1)\right)$$

   - by setting, on the iteration *i+1*, the vector $\Theta$ to its value calculated on the iteration *i*, and by solving the optimization problem according to the equations:

$$\left(\widehat{\boldsymbol{B}}(i+1), \widehat{\boldsymbol{\Delta}}(i+1)\right) = arg \min_{B(i+1),\Delta(i+1)} D\left(\boldsymbol{I}, \boldsymbol{M}(i+1)\right)$$

$$\forall n \in [1,N],\ M_n(i+1) = \theta_n(i).\left(\boldsymbol{X}_n.\boldsymbol{B}(i+1) + \boldsymbol{Y}_n.\boldsymbol{\Delta}(i+1)\right)$$

9. The method according to claim 8, **characterized in that** the first iteration is performed by setting $\forall n \in [1,N], X_n.B(1) + Y_n.\Delta(1) = 1$

10. A computer program product stored on a computer-readable computing medium comprising instructions for the execution of a method according to any of the preceding claims.

11. A method for fabricating a DNA chip comprising copies of a DNA strand, or probe, capable of forming a hybrid of length $L_{bp}$ with a target strand of nucleic acid, in particular of DNA, of length greater than $L_{bp}$ without mismatch, said method consisting in:

    - identifying a set of portions of length $L_{bp}$ on the target DNA strand;
    - for each identified portion of the target DNA strand, or "candidate target":

      ◦ determining the complementary DNA strand, or "candidate probe", and calculating a first affinity $\phi$ of the candidate probe and target by implementing a method according to any of claims 1 to 9;
      ◦ calculating a second affinity $\phi$ of the candidate probe with each element of a set of nucleic stands not comprising the candidate target by implementing a method according to any of claims 1 to 9;

- selecting, from the determined candidate probes, at least one probe

  ◦ the first affinity $\phi$ is above a predetermined first threshold $S_1$; and
  ◦ each of the second affinities $\phi$ is below a second threshold $S_2$ strictly lower than the first threshold $S_1$;

- fabricating the DNA chip with each selected candidate probe.

12. The method for fabricating a DNA chip according to claim 11, consisting in selecting, from determined candidate probes, at least one probe for which at most N calculated affinities are above a predetermined threshold and for which the other second calculated affinities are below a second threshold strictly lower than the first threshold.

EP 3 227 813 B1

10

fabrication
puce à ADN

29

génération
non spécifique

30

génération
sondes
candidates

32

génération
alignements

34

modélisation
hybrides

36

Calcul
affinité
Φ

38

sélection
sonde(s)
puce à
ADN

12

banque
de
transcrits

14

transcrit
cible

20

transcrits
non
spécifiques

22

sondes
candidates

24

hybrides
transcrits
-
sondes
candidates

26

x,y

16

$\tilde{\beta}, \tilde{\delta}$

28

$\Phi_1$
$\Phi_2$

18

$S_1$
$S_2$

**Fig. 1**

Fig. 2

transcrit 42

sonde candidate 40

44

portion 46 du transcrit 42

| 1 | 2 | 3 | 4 | 5 | 6 | 7 | 8 |

| 21 | 22 | 23 | 24 | 25 | — base

1 2 3 4 5 6 7 8

21 22 23 24 25 — base

sonde candidate 40

$L_{bp} = 25$

Fig. 3

EP 3 227 813 B1

**Fig. 4**

**Fig. 5**

**Fig. 6**

transcrit cible

sondes candidates

**Fig. 7**

Fig. 8

Fig. 9

EP 3 227 813 B1

72 — sélection
sondes spécifiques
$\{SA_{PM}\}$

70 —

74 — dégénérescence
sondes $\{SA_{PM}\}$
$\rightarrow \{SA_{MM}\}$

76 — fabrication
puce à ADN
$\{SA_{PM}\},\{SA_{MM}\}$

78 — mesure intensités
$\{SA_{PM}\},\{SA_{MM}\}$
transcrites cibles

80 — filtrage
réactions croisées

82 —
84 — génération :
intensités d'apprentissage
intensités de validation

86 — modélisation de
chaque hybride
sondes / transcrit

88 — Calcul $M / I - \tilde{\sigma M}$ minimum
avec intensités d'apprentissage

90 — validation $\tilde{M}$
avec intensités
de validation

# Fig. 10

2-mers

Nombre de divisions

**Fig. 11A**

3-mers

R^2

Nombre de divisions

**Fig. 11B**

4-mers

Nombre de divisions

**Fig. 11C**

5-mers

R^2

Nombre de divisions

**Fig. 11D**

EP 3 227 813 B1

**Fig. 12**

Fig. 13B

Fig. 13A

EP 3 227 813 B1

Puce V2
R^2=0.55

Puce V3
R^2=0.40

intésités mesurées (log 2(intensité))

inténsités prédites
(log 2(intensité))

**Fig. 14A**

intesités mesurées (log 2(intensité))

inténsités prédites
(log 2(intensité))

**Fig. 14B**

R2 affinity

V2          V3

**Fig. 14C**

| | Distribution des facteurs confondants | Coefficients de variation | Titration monotone |
|---|---|---|---|
| Niveau sonde | Fig. 15A | Fig. 15C | Fig. 15E |
| Niveau probeset | Fig. 15B | Fig. 15D | Fig. 15E |

EP 3 227 813 B1

**Fig. 15G**

**Fig. 16A**

**Fig. 16B**

**Fig. 16C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MEI et al.** Probe selection for high-density oligonucleotide arrays. *Proceedings of the National Academy of Sience,* Septembre 2003, vol. 100 (20), 11237-11242
- **ZHANG et al.** A model of molecular interactions on short oligonucleotide microarrays. *Nature biotechnology,* Juillet 2003, vol. 21 (7), 818-821
- **NAOAKI ONO et al.** An improved physico-chemical model of hybridization on high-density oligonucleotide microarrays. *Bioinformatics,* 2008, vol. 24 (10
- **GIMENEZ et al.** Custom human endogenous retroviruses dedicated microarray identifies self-induced HERV-Wfamily elements reactivated in testicular cancer upon methylation control. *Nucleic Acids Research,* Avril 2010, vol. 38 (7), 2229-2246
- **LI H. et al.** Fast and accurate long-read alignment with Burrows-Wheeler transform. *Bioinformatices,* vol. 26 (5), 589-595
- **LI et al.** Model-based analysis ofoligonucleotide arrays : expression index computation and oulier detection. *Proceedings of the National Academy of Science,* Novembre 2006, vol. 98 (1), 31-36
- **PÉROT et al.** Microarray-based sketches of the HERV transcriptome landscape. *PLoS One, 2012,* Juin 2012, vol. 7 (6), e40194
- **LI ; WONG.** Model-based analysis of oligonucleotide arrays : Expression index computation and outlier detection. *Proceedings of the National Academy of Sciences,* 2001, vol. 98 (1), 31-36
- **IRIZARRY et al.** *Exploration, normalization, and summaries of high density oligonucleotide array probe level data, Biostatistics,* Avril 2003, vol. 4 (2), 249-64
- The MicroArray Quality Control (MAQC) project shows inter- and intraplatform reproducibility of gene expression measurements. *Nature Biotechnology,* Septembre 2006, vol. 24 (9), 1151-61
- **TUSHER VG ; TIBSHIRANI R ; CHU G.** Significance analysis of microarrays applied to the ionizing radiation response. *Proceedings of the National Academy of Sciences of the USA.,* Avril 2001, vol. 24;98 (9), 5116-21